# EUROPEAN PATENT SPECIFICATION

(11) **EP 4 402 228 B1**
(45) Date of publication and mention of the grant of the patent: **16.04.2025**
(21) Application number: 22785952.7
(22) Date of filing: 12.09.2022
(51) Int. Cl.: C10L 3/10

(54) **HYDROGEN SULPHIDE AND MERCAPTANS SCAVENGERS**
SCHWEFELWASSERSTOFF- UND MERCAPTANFÄNGER
FIXATEURS DE SULFURE D'HYDROGÈNE ET DE MERCAPTANS

(30) Priority: 13.09.2021 EP 21306252
(43) Date of publication of application: 24.07.2024
(73) Proprietor: TotalEnergies OneTech, 92400 Courbevoie (FR)
(72) Inventor: SUNANDA MONDKAR, Hemant, Pune, Maharashtra 411028 (IN); TORT, Frédéric, 69530 Brignais (FR); REDDY, Vishnu Vardhan, Telangana 508213 (IN)
(74) Representative: August Debouzy
(86) International application number: PCT/EP2022/075226
(87) International publication number: WO 2023/036969

(56) References cited:
- GB-A- 2 586 594
- US-A1- 2021 198 129

## Description

### TECHNICAL FIELD

The present invention pertains to novel hydrogen sulphide and mercaptans scavengers and to compositions containing the scavengers. The present invention also pertains to the use of the scavengers for scavenging hydrogen sulphide and mercaptans in hydrocarbon containing compositions and in water-containing compositions. The present invention also relates to a method for scavenging hydrogen sulphide and/or mercaptans comprising contacting a media such as crude oils, fuels or natural gas or drilling fluids with the scavenging composition of the invention.

### BACKGROUND OF THE INVENTION

Hydrogen sulphide is a colourless and fairly toxic, flammable and corrosive gas which also has a characteristic odour at a very low concentration. Hydrogen sulphide dissolves in hydrocarbon and water streams and is also found in the vapour phase above these streams and in natural gas. In drilling subterranean wells, notably those for oil or gas, hydrogen suphide can be present in substantial amounts. The drilling fluid may drive the hydrogen sulphide to the surface.

The hydrogen sulphide emissions can therefore be a nuisance to workers operating in the production, transport, processing and storage of oil products such as crude oil, asphalt, Liquid Petroleum Gas, Heavy Fuel Oil, gasoline, kerosene, and diesel fuel. Hydrogen sulphide may also react with hydrocarbon components present in fuel. It would therefore be desirable for the workers' comfort and safety to reduce or even eliminate the hydrogen sulphide emissions during the manipulation of said products.

Legislations have been in place for years, imposing strict regulations on hydrogen sulphide levels of hydrocarbon containing streams in pipelines, in storage and in shipping containers. A variety of chemical scavengers are available to reduce both the concentration and corresponding hazard of hydrogen sulphide in produced gas, crude oil and refined products. The three major classes of hydrogen sulphide scavengers used for the above-mentioned products are water soluble scavengers, oil soluble scavengers and metal-based scavengers.

Current widely used chemistry based on N-alkyl-triazine (MMA-Triazine and MEA-Triazine) has its own limitations in applications. US 2021/198129 and GB 2 586 594 disclose scavengers based on MEA-Triazine. These products are produced in water-based formulations and has limitations in attaining their highest active content close to 100%. Also, on reaction with hydrogen sulfide gas they produce methylamine and monoethanolamine as side products, respectively. Out of which Methylamine is a gaseous, flammable, highly corrosive with low flash point (-30°C), low molecular amine, it also causes topline corrosion.

MEA-Triazine is identified with high risk of fouling and categorized as fatal by inhalation within the oil and gas industry.

There is a need for a hydrogen sulphide and/or mercaptan scavenger having a similar or even a better scavenging efficiency than conventional symmetric triazine, such as MEA-triazine, but with a reduced or zero risk of fouling.

### SUMMARY OF THE INVENTION

The present invention is directed to a compound of formula (I): 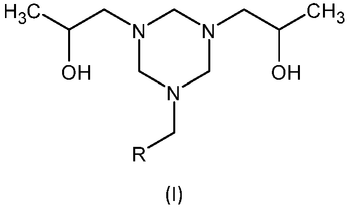 wherein R is a radical selected from a linear, branched or cyclic hydrocarbyl group optionally comprising one or more heteroatoms, such as oxygen, sulfur, halogen, silicon, nitrogen atoms.

According to an embodiment, R is selected from: R¹-OH, R¹-[O-R²]ₚ-OH, optionally substituted benzyl, R¹-NR³R⁴, R⁵, R¹-[N(H)-R²]ₙ-NR³R⁴, R¹-[O-R²]ₚ-NR³R⁴, R¹-Q, R¹-[N(H)-R²]ₙ-Q, R¹-[O-R²]ₚ-Q, R¹-N(R³-Q)(R⁴-Q) wherein:
- R¹ is selected from a linear, branched or cyclic alkyl or alkenyl radical having from 1 to 24 carbon atoms, preferably from 1 to 16 carbon atoms, more preferably from 1 to 12 carbon atoms, even more preferably from 1 to 8 carbon atoms or from an aromatic radical optionally substituted by one or more substituent selected from C₁-C₆-alkyl, hydroxyl, C₁-C₆-alkoxy, or C₁-C₆-alkylamine, preferably a phenyl radical substituted by C₁-C₆-alkyl;
- R² is a linear, branched or cyclic alkyl or alkenyl radical having from 1 to 24 carbon atoms, preferably from 1 to 16 carbon atoms, more preferably from 1 to 12 carbon atoms, even more preferably from 1 to 8 carbon atoms;
- R³ and R⁴ are, independently to each other, a hydrogen atom or a linear, branched or cyclic alkyl or alkenyl radical having from 1 to 16 carbon atoms, preferably from 1 to 12 carbon atoms, more preferably from 1 to 8 carbon atoms, even more preferably from 1 to 4 carbon atoms;
- R⁵ is a linear, branched or cyclic alkyl or alkenyl radical having from 1 to 30 carbon atoms, preferably from 1 to 24 carbon atoms, more preferably from 1 to 16 carbon atoms, even more preferably from 1 to 12 carbon atoms;
- Q is a radical selected from morpholine, piperidine, piperazine, or the monovalent radical of formula: 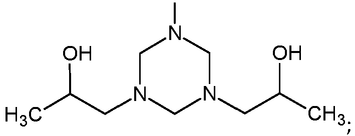
- the optionally substituted benzyl can be a benzyl substituted by one or more substituent selected from C₁-C₆-alkyl, hydroxyl, C₁-C₆-alkoxy, C₁-C₆-alkylamine;
- n is an integer ranging from 1 to 10, preferably from 1 to 6, more preferably from 1 to 4, even more preferably from 1 to 2;
- p is an integer ranging from 1 to 10, preferably from 1 to 6, more preferably from 1 to 4, even more preferably from 1 to 2.

According to an embodiment, R is selected from: R¹-OH, R¹-O-R²-OH, benzyl, R¹-NR³R⁴, R⁵, R¹-N(H)-R²-NR³R⁴, R¹-O-R²-O-R²-NR³R⁴, R¹-Q, R¹-N(H)-R²-Q, R¹-O-R²-O-R²-Q, R¹-N(R³-Q)₂ wherein:
- R¹ is selected from a linear or branched alkyl radical having from 1 to 16 carbon atoms, preferably from 1 to 12 carbon atoms, more preferably from 1 to 8 carbon atoms, even more preferably from 1 to 4 carbon atoms or from a phenyl radical substituted by C₁-C₆-alkyl;
- R² is a linear or branched alkyl radical having from 1 to 16 carbon atoms, preferably from 1 to 12 carbon atoms, more preferably from 1 to 8 carbon atoms, even more preferably from 1 to 4 carbon atoms;
- R³ and R⁴ are, independently to each other, a hydrogen atom or a linear or branched alkyl radical having from 1 to 16 carbon atoms, preferably from 1 to 12 carbon atoms, more preferably from 1 to 8 carbon atoms, even more preferably from 1 to 4 carbon atoms;
- R⁵ is a linear or branched alkyl radical having from 1 to 24 carbon atoms, preferably from 1 to 16 carbon atoms, more preferably from 1 to 12 carbon atoms, even more preferably from 1 to 8 carbon atoms;
- Q is a morpholine radical or the monovalent radical of formula:

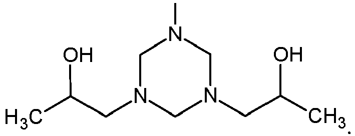

According to an embodiment, R is selected from: R¹-OH, R¹-O-R²-OH, benzyl, R¹-NR³R⁴, R⁵, R¹-N(H)-R²-NR³R⁴, R¹-O-R²-O-R²-NR³R⁴, R¹-Q, R¹-N(H)-R²-Q, R¹-O-R²-O-R²-Q, R¹-N(R³-Q)₂ wherein:
- R¹ is a linear alkyl radical having from 1 to 4 carbon atoms;
- R² is a linear alkyl radical having from 1 to 4 carbon atoms;
- R³ and R⁴ are, independently to each other, a hydrogen atom or a linear alkyl radical having from 1 to 4 carbon atoms;
- R⁵ is a linear alkyl radical having from 1 to 8 carbon atoms;
- Q is a morpholine radical or the monovalent radical:

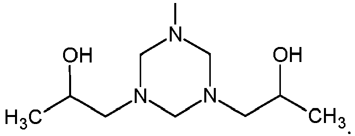

According to an embodiment, the compound of the invention replies to one of the following formula:

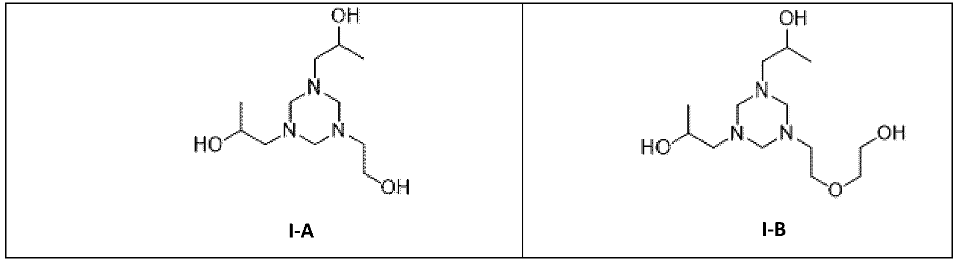

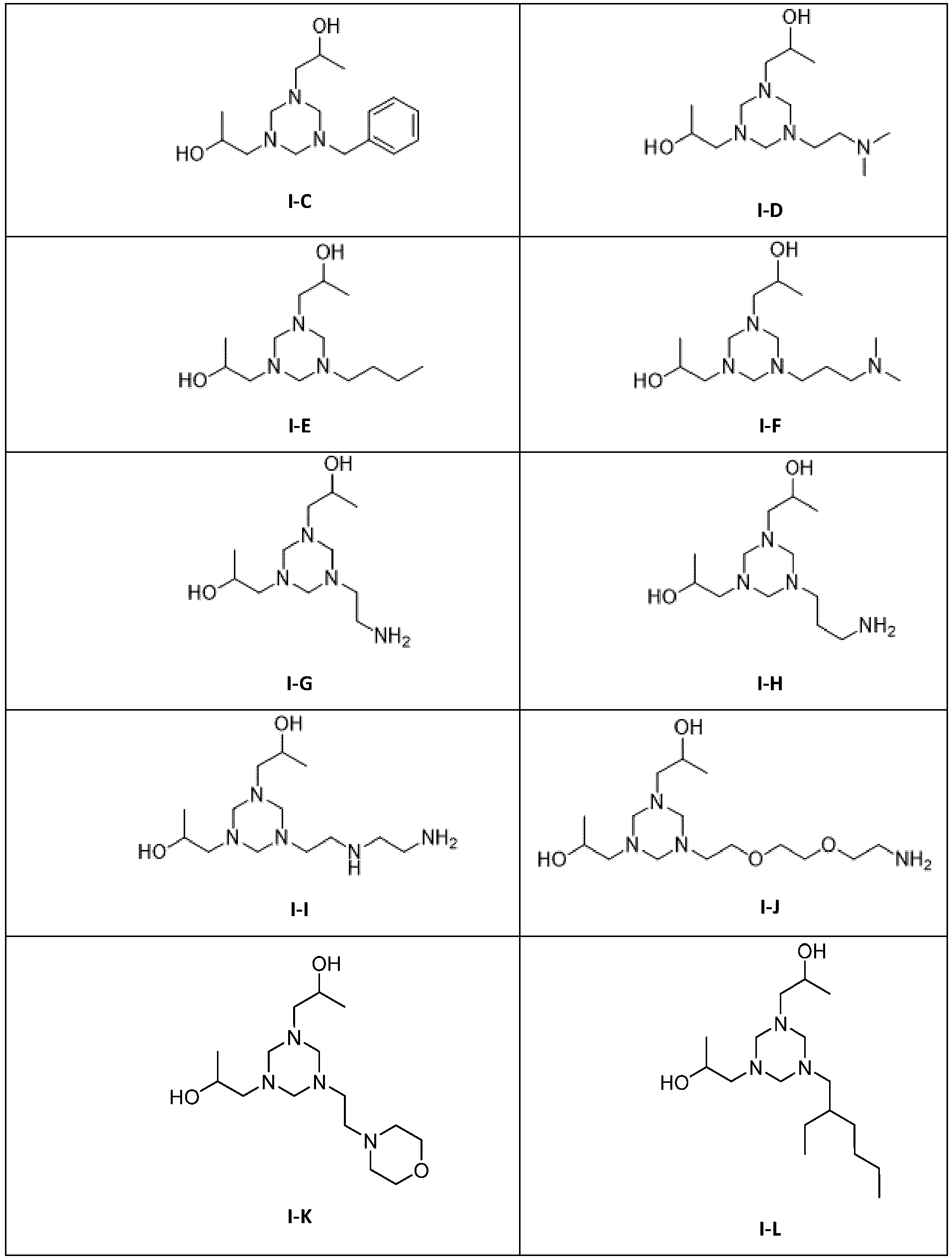

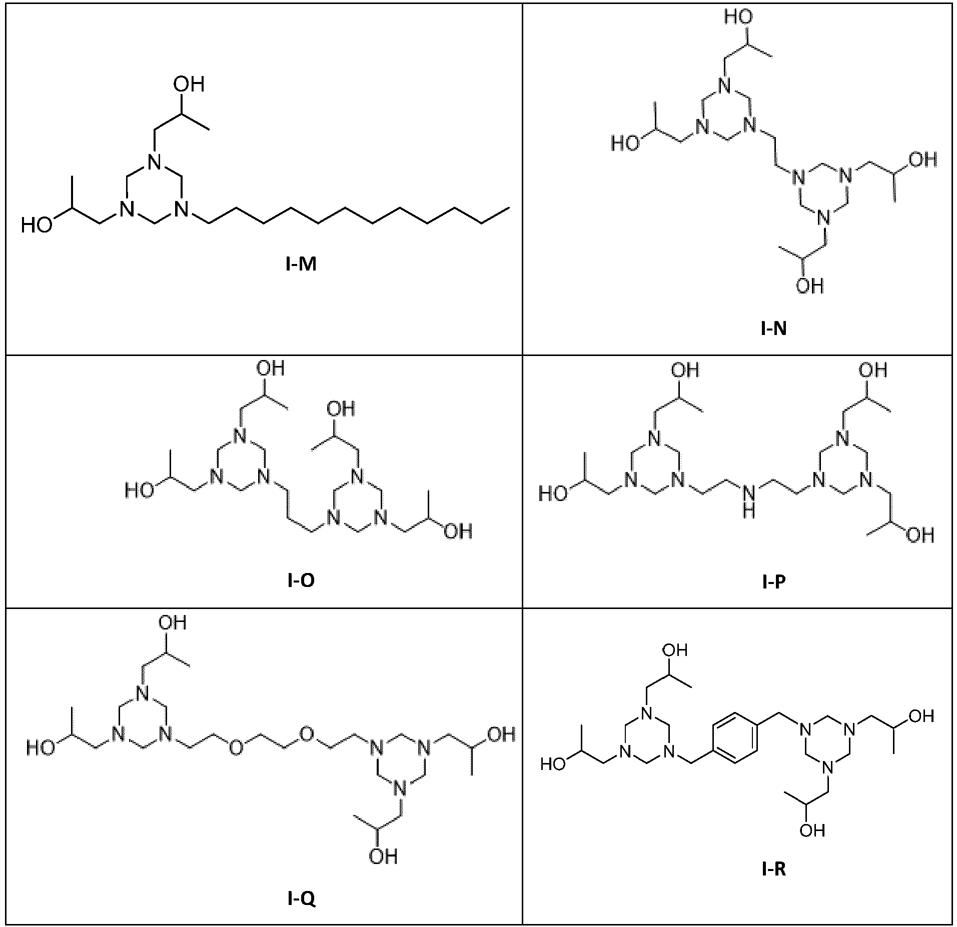

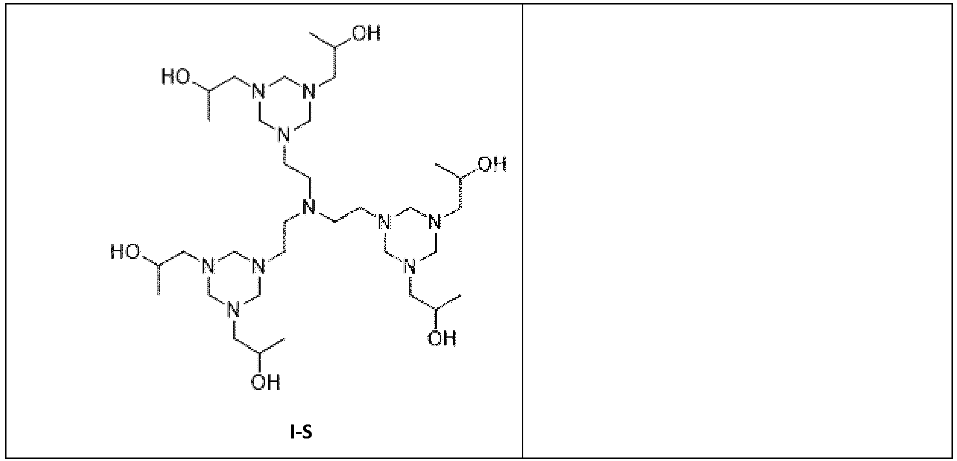

The present invention is also directed to a process for manufacturing the compound according to the invention, comprising a step of contacting at least one bis(5-methyloxazolidin-3-yl)methane (BMOM) with at least one primary amine.

According to an embodiment, the contacting step is performed with a molar ratio BMOM:primary amines ranging from 1:5 to 5:1, preferably from 1:1 to 3:1.

The present invention is also directed to a composition comprising:
- from 20 to 98%wt of at least one compound according to any one of claims 1 to 5, and
- from 2 to 80%wt of solvent(s), based on the total weight of the composition.

The present invention is also directed to the use of the compound according to the invention or of the composition according to the invention, to scavenge hydrogen sulphide and mercaptans in streams selected from hydrocarbon streams and water-containing streams.

According to an amendment of the use of the invention, the compound is added in an amount of at least 1%wt, based on the total weight of hydrogen sulphide and mercaptans in the stream.

According to an amendment of the use of the invention, the streams are selected from hydrocarbon-containing streams such as liquefied petroleum gas (LPG), finished fuels, crude and heavy residual oils, natural gas asphalt, oil-based drilling fluids, and water-containing streams such as water-based drilling fluids.

The present invention is also directed to a hydrocarbon-containing composition comprising hydrocarbons and the compound according to the invention or of the composition according to the invention.

The present invention is also directed to a water-containing stream comprising water and the compound according to the invention or of the composition according to the invention.

Unlike conventional symmetric triazines, such as MEA-triazine, the scavenging compositions according to the invention comprising these asymmetric triazines will not tend to produce solid reaction by-products. In addition, the scavenger of the invention will have a minimal impact on pH, which reduces associated mineral scaling.

The compounds of the invention can be used at low temperatures, where the temperatures in winter may fall below -40°C, for example in hydrocarbon reserves in the northern hemisphere, like, northern USA, Canada, Russia, Kazakhstan, Northern China and Norway.

On the contrary, traditional symmetrical triazines of the prior art have a freezing point near to these application temperatures, such that they cannot be injected into the hydrocarbon streams using typical methods. In these areas the symmetrical triazines of the prior art are diluted with expensive and flammable solvents and antifreezes, such as methanol or ethylene glycol, in order to prevent freezing and low temperature handling capability. This dilution limits H₂S scavenging and also increases the cost of the product.

The compounds of the invention are beneficial due to their lower freezing points below -40°C or even below -45°C. This allows the compounds of the invention to be used for H₂S scavenging at low application temperatures without adding any expensive and flammable solvents and antifreezes.

One additional advantage of the compounds of the invention is that they can be obtained as 100% active products. On the contrary, other triazine compounds of the prior art, such as MEA Triazine, always contains water.

The compounds of the invention, as water-free products, can be employed in applications where water cannot be part of the formulations. As an example, the compounds of the invention can be used as H₂S scavengers for fuels, where water is avoided.

One additional advantage of the compounds of the invention is that they are soluble in various organic solvents, making them suitable in solvent-based systems.

### BRIEF DESCRIPTION OF THE FIGURES

Fig. 1 represents the amount of hydrogen sulphide in ppm in function of the time during the test for different scavenging compositions.
Fig. 2 represents the amount of hydrogen sulphide in ppm in function of the time during the test for different scavenging compositions.
Fig. 3 represents the amount of hydrogen sulphide in ppm in function of the time during the test for different scavenging compositions.

### DETAILED DESCRIPTION OF THE INVENTION

The present invention is directed to a compound having the general formula (I): 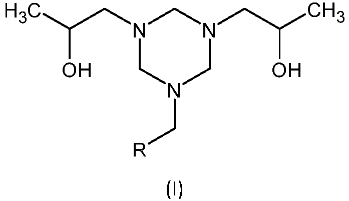 Wherein:
R is a radical selected from a linear, branched or cyclic hydrocarbyl group optionally comprising one or more heteroatoms, such as oxygen, sulfur, halogen, silicon, nitrogen atoms.

According to the present invention, R is typically different from CH(CH₂OH).

Within the meaning of the present invention, a "hydrocarbyl group" is a linear, branched or cyclic group that can be aliphatic or aromatic and when it is a cyclic group, the cycle(s) can be substituted by linear or branched groups. The hydrocarbyl group comprises carbon atoms and hydrogen atoms and optionally heteroatoms selected from oxygen, sulfur, halogen, silicon, nitrogen, preferably selected from oxygen, sulfur, halogen, silicon.

According to an embodiment, R is selected from: R¹-OH, R¹-[O-R²]ₚ-OH, optionally substituted benzyl, R¹-NR³R⁴, R⁵, R¹-[N(H)-R²]ₙ-NR³R⁴, R¹-[O-R²]ₚ-NR³R⁴, R¹-Q, R¹-[N(H)-R²]ₙ-Q, R¹-[O-R²]ₚ-Q, R¹-N(R³-Q)(R⁴-Q) wherein:
- R¹ is selected from a linear, branched or cyclic alkyl or alkenyl radical having from 1 to 24 carbon atoms, preferably from 1 to 16 carbon atoms, more preferably from 1 to 12 carbon atoms, even more preferably from 1 to 8 carbon atoms or from an aromatic radical optionally substituted by one or more substituent selected from C₁-C₆-alkyl, hydroxyl, C₁-C₆-alkoxy, or C₁-C₆-alkylamine, preferably a phenyl radical substituted by C₁-C₆-alkyl;
- R² is a linear, branched or cyclic alkyl or alkenyl radical having from 1 to 24 carbon atoms, preferably from 1 to 16 carbon atoms, more preferably from 1 to 12 carbon atoms, even more preferably from 1 to 8 carbon atoms;
- R³ and R⁴ are, independently to each other, a hydrogen atom or a linear, branched or cyclic alkyl or alkenyl radical having from 1 to 16 carbon atoms, preferably from 1 to 12 carbon atoms, more preferably from 1 to 8 carbon atoms, even more preferably from 1 to 4 carbon atoms;
- R⁵ is a linear, branched or cyclic alkyl or alkenyl radical having from 1 to 30 carbon atoms, preferably from 1 to 24 carbon atoms, more preferably from 1 to 16 carbon atoms, even more preferably from 1 to 12 carbon atoms;
- Q is a radical selected from morpholine, piperidine, piperazine, or the monovalent radical of formula: 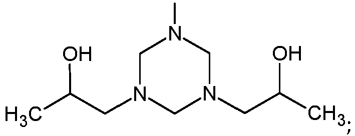
- the optionally substituted benzyl can be a benzyl substituted by one or more substituent selected from C₁-C₆-alkyl, hydroxyl, C₁-C₆-alkoxy, C₁-C₆-alkylamine;
- n is an integer ranging from 1 to 10, preferably from 1 to 6, more preferably from 1 to 4, even more preferably from 1 to 2;
- p is an integer ranging from 1 to 10, preferably from 1 to 6, more preferably from 1 to 4, even more preferably from 1 to 2.

According to an embodiment, R is selected from: R¹-OH, R¹-O-R²-OH, optionally substituted benzyl, R¹-NR³R⁴, R⁵, R¹-N(H)-R²-NR³R⁴, R¹-O-R²-O-R²-NR³R⁴, R¹-Q, R¹-N(H)-R²-Q, R¹-O-R²-O-R²-Q, R¹-N(R³-Q)₂ wherein:
- R¹ is selected from a linear, branched or cyclic alkyl or alkenyl radical having from 1 to 24 carbon atoms, preferably from 1 to 16 carbon atoms, more preferably from 1 to 12 carbon atoms, even more preferably from 1 to 8 carbon atoms or from a phenyl radical substituted by C₁-C₆-alkyl;
- R² is a linear, branched or cyclic alkyl or alkenyl radical having from 1 to 24 carbon atoms, preferably from 1 to 16 carbon atoms, more preferably from 1 to 12 carbon atoms, even more preferably from 1 to 8 carbon atoms;
- R³ and R⁴ are, independently to each other, a hydrogen atom or a linear, branched or cyclic alkyl or alkenyl radical having from 1 to 16 carbon atoms, preferably from 1 to 12 carbon atoms, more preferably from 1 to 8 carbon atoms, even more preferably from 1 to 4 carbon atoms;
- R⁵ is a linear, branched or cyclic alkyl or alkenyl radical having from 1 to 30 carbon atoms, preferably from 1 to 24 carbon atoms, more preferably from 1 to 16 carbon atoms, even more preferably from 1 to 12 carbon atoms;
- Q is a morpholine radical or the monovalent radical of formula: 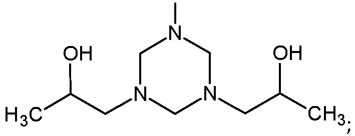
- the optionally substituted benzyl can be a benzyl substituted by one or more substituent selected from C₁-C₆-alkyl, hydroxyl, C₁-C₆-alkoxy, C₁-C₆-alkylamine.

According to an embodiment, R is selected from: R¹-OH, R¹-O-R²-OH, benzyl, R¹-NR³R⁴, R⁵, R¹-N(H)-R²-NR³R⁴, R¹-O-R²-O-R²-NR³R⁴, R¹-Q, R¹-N(H)-R²-Q, R¹-O-R²-O-R²-Q, R¹-N(R³-Q)₂ wherein:
- R¹ is selected from a linear or branched alkyl radical having from 1 to 16 carbon atoms, preferably from 1 to 12 carbon atoms, more preferably from 1 to 8 carbon atoms, even more preferably from 1 to 4 carbon atoms or from a phenyl radical substituted by C₁-C₆-alkyl;
- R² is a linear or branched alkyl radical having from 1 to 16 carbon atoms, preferably from 1 to 12 carbon atoms, more preferably from 1 to 8 carbon atoms, even more preferably from 1 to 4 carbon atoms;
- R³ and R⁴ are, independently to each other, a hydrogen atom or a linear or branched alkyl radical having from 1 to 16 carbon atoms, preferably from 1 to 12 carbon atoms, more preferably from 1 to 8 carbon atoms, even more preferably from 1 to 4 carbon atoms;
- R⁵ is a linear or branched alkyl radical having from 1 to 24 carbon atoms, preferably from 1 to 16 carbon atoms, more preferably from 1 to 12 carbon atoms, even more preferably from 1 to 8 carbon atoms;
- Q is a morpholine radical or the monovalent radical of formula:

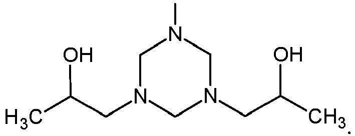

Within the meaning of the present invention, an alkyl group is a saturated group consisting in carbon atoms and hydrogen atoms, that can be linear, branched or cyclic.

Within the meaning of the present invention, an alkenyl group is an unsaturated group consisting in carbon atoms and hydrogen atoms, that can be linear, branched or cyclic. The alkenyl group can be mono-unsaturated or poly-unsaturated.

According to a preferred embodiment, R³ is identical to R⁴.

According to an embodiment, the compound is selected from the following compounds of table 1:

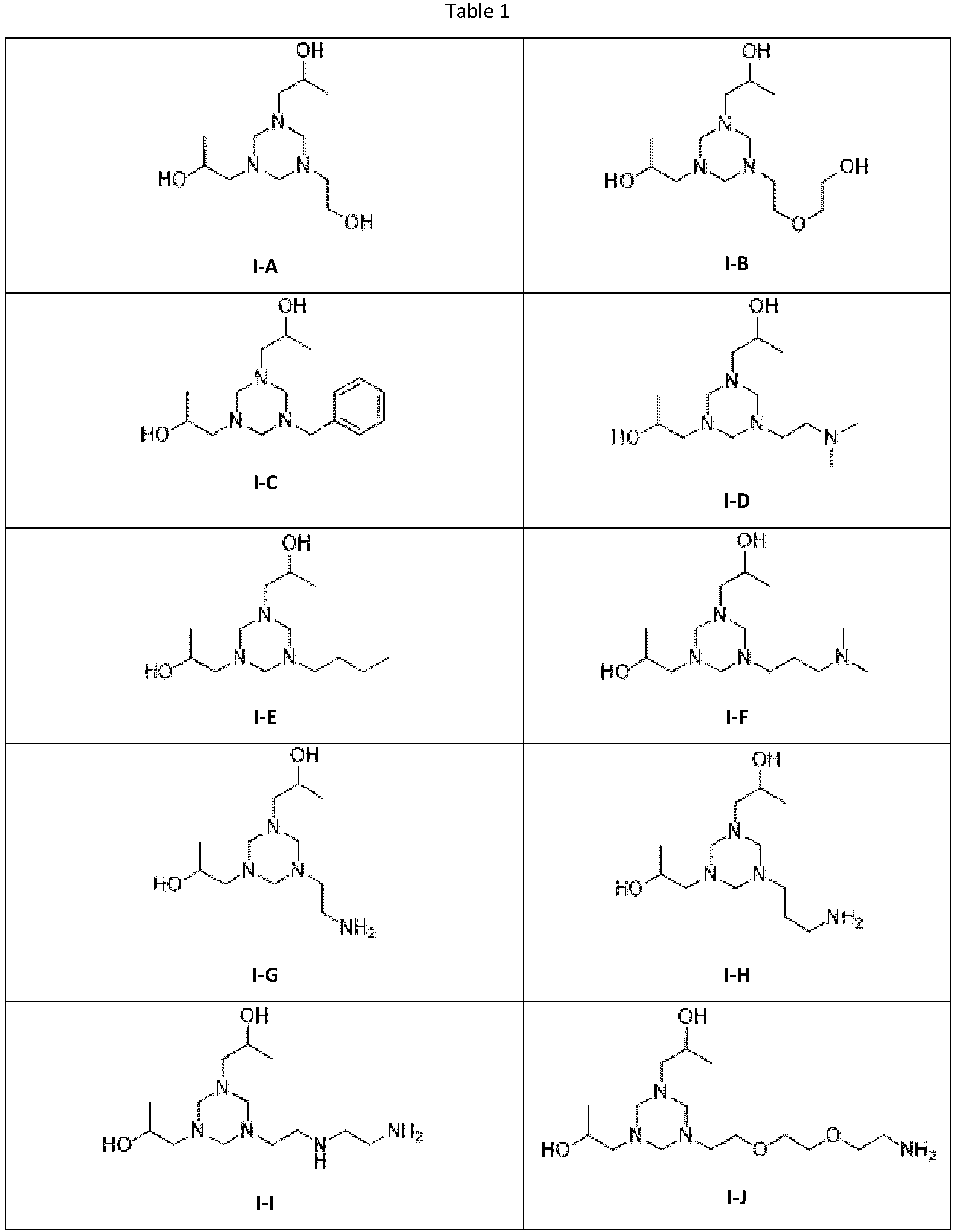

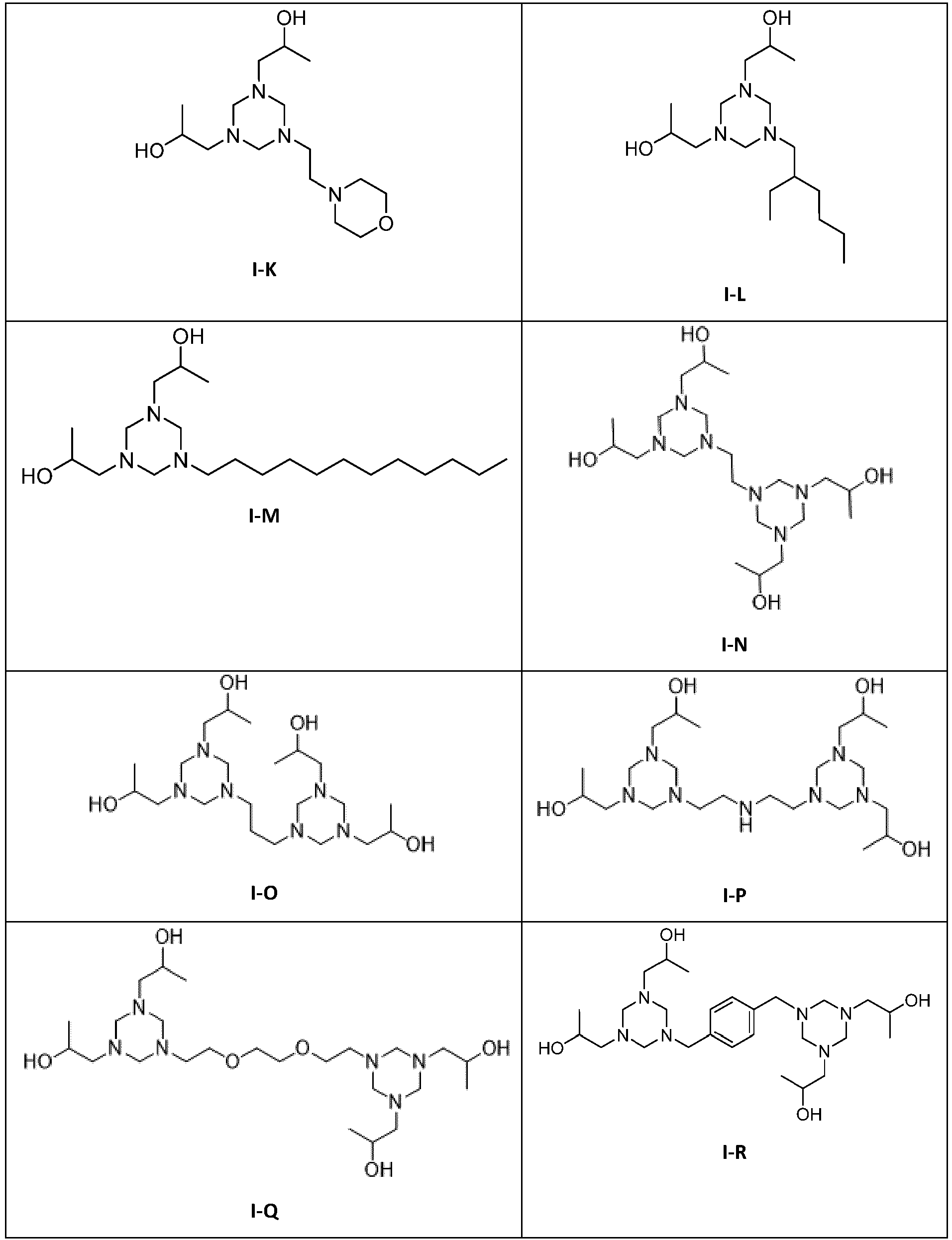

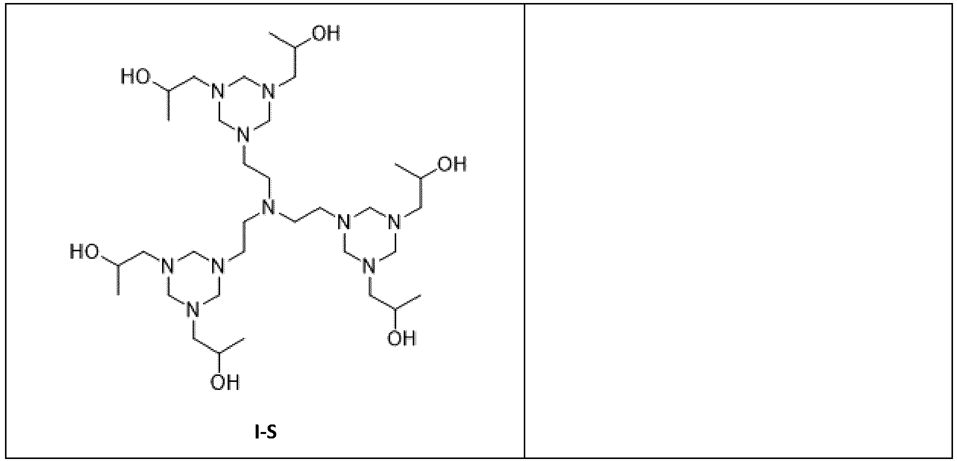

According to an embodiment, the compound of the invention is selected from compound I-A, compound I-C, compound I-E-, compound I-F, compound I-G, compound I-I, compound I-K, compound I-L, compound I-M, compound I-O, compound I-P, compound I-Q, compound I-R or compound I-S.

Another object of the present invention is a process for manufacturing the compound of the invention.

The compound of the invention can be obtained by contacting bis(5-methyloxazolidin-3-yl)methane (BMOM) with primary amines, wherein: BMOM has the formula

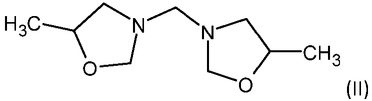

BMOM is also named 3,3'-methylenebis(5-methyloxazolidine) known as the MBO.

Primary amines have the formula (III): NH₂-CH₂-R, wherein R is as defined above in formula (I).

The molar ratio BMOM:primary amines can range from 1:5 to 5:1, preferably from 1:1 to 3:1.

As an example, compounds I-A to I-J can be obtained with a molar ratio BMOM:primary amines of 1:1; compounds I-K to I-N can be obtained with a molar ratio BMOM:primary amines of 2:1 and compound I-O can be obtained with a molar ratio BMOM:primary amines of 3:1.

According to an embodiment, primary amines may be selected from monoethanolamine (MEA), aminoethylethanolamine (AEEA), benzylamine (BA), N,N-dimethylethylenediamine (DEDA), n-butylamine (NBA), N,N-dimethylpropane-1,3-diamine (DMAPA), 1,2-ethylenediamine (EDA), 1,3-propyleneamine (PDA), diethylenetriamine (DETA), Jeffamine^{®} EDR-148 (EDR-148), 4-(2-Aminoethyl)morpholine (AEM), 2-ethyl-1-hexylamine (2EHA), 1-Dodecylamine (DDA), diethylenetriamine (DETA), p-xylylenediamine (XLDA), tris(2-aminoethyl)amine (TAEA), and mixtures thereof, preferably from monoethanolamine (MEA), benzylamine (BA), n-butylamine (NBA), N,N-dimethylpropane-1,3-diamine (DMAPA), 1,2-éthylenediamine (EDA), diethylenetriamine (DETA), Jeffamine^{®} EDR-148 (EDR-148), 4-(2-Aminoethyl)morpholine (AEM), 2-ethyl-1-hexylamine (2EHA), 1-Dodecylamine (DDA), diethylenetriamine (DETA), p-xylylenediamine (XLDA), tris(2-aminoethyl)amine (TAEA), and mixtures thereof.

Examples of synthesis of the compound of the invention are given in the experimental part.

The compound of the invention can be used to scavenge hydrogen sulphide and/or mercaptans in hydrocarbons. The compound of formula (I) is also named "H₂S scavenger" in the present invention.

The compound can be added into hydrocarbons directly as 100% active matter or via a composition, named a scavenging composition, comprising said compound. According to an embodiment, the composition according to the invention comprises at least 5%wt, preferably at least 20%wt, more preferably at least 40%wt, of the compound as defined in the present invention, based on the total weight of the composition.

According to an embodiment, the composition according to the invention comprising the compound defined above, further comprises one or more solvents.

According to an embodiment, the composition comprises:
- from 20 to 98%wt, preferably from 25 to 90%wt, more preferably from 30 to 80%wt, of the compound defined in the present invention, and
- from 2 to 80%wt, preferably from 10 to 75%wt, more preferably from 20 to 70%wt, of solvent(s), based on the total weight of the composition.

The solvent can be selected in order to have a solution or a dispersion of the compound of the invention. The solvent can thus be either oil soluble, or water soluble or the solvent can have a dual solubility.

Preferably, the solvent is selected from water, methanol, poly alkyl ethers, aliphatic or aromatic solvents, such as N-methylpyrrolidone, butyl carbitol, xylene, toluene, and benzene. Typically, the solvent does not allow to scavenge or neutralize hydrogen sulphide or mercaptans in hydrocarbon streams. However, depending on the final use of the scavenging composition, a solvent having a dual solubility, i.e. a water solubility and a solubility in hydrocarbons, can be preferred. Butyl carbitol is a suitable solvent since it has this dual solubility.

Within the meaning of the present invention, a product is said "soluble" in water or water-soluble if it can form a solution in water. A product is said "not-soluble" in water if it can form a dispersion in water.

The scavenging compositions according to the invention can be used in both water soluble and oil-soluble solvents.

Depending on the media in which they are intended to be used, the compound of the invention can be dissolved in adapted solvents. The media can be hydrocarbons or water-containing compositions such as water-based muds.

Hydrocarbons can be selected from liquefied petroleum gas (LPG), finished fuels such as diesel fuel, kerosene or gasoline, crude and heavy residual oils, and asphalt.

Once the H₂S scavenger(s) is/are added into hydrocarbons, a hydrocarbon-containing composition is obtained. The hydrocarbon-containing composition can be either a single-phase hydrocarbon composition or a multiphase system comprising oil/water or oil/water/gas or gas/water.

In multiphase systems comprising water, it is preferred to use a water-soluble scavenger dissolved in a water-soluble solvent. Examples of water-soluble solvents include water, glycols, butylcarbitol. Water-soluble scavengers are among the most common scavengers and are often the product of choice for applications at temperatures below 93°C. Economical costs and fast reaction rates make them attractive options. Water soluble scavengers are preferred for use in LPG, residues and crude oils and for use in water-based muds.

Preferably, the scavenging composition comprises:
- from 20 to 98%wt of compound(s) of formula (I), preferably from 25 to 70%wt of compound(s) of formula (I), more preferably from 30 to 60%wt of compound(s) of formula (I), and
- from 2 to 80%wt of solvent(s), preferably from 30 to 75%wt of solvent(s), more preferably from 30 to 60%wt of solvent(s),
based on the total weight of the scavenging composition.

### Hydrocarbon-containing composition

The invention is also directed to a hydrocarbon-containing composition comprising:
- at least 70%wt of hydrocarbon(s), and
- at least 10 ppm by weight of compound(s) as defined in the invention,
- optionally at least 10 ppm by weight of one or more solvent(s) different from the hydrocarbon(s).

Preferably, the compound comprises one or more of the features defined above in relation to the composition of the invention.

Preferably, the solvent comprises one or more of the features defined above in relation to the composition of the invention.

The hydrocarbon-containing composition can be prepared by adding the scavenging composition of the invention comprising the compound alone or in the presence of solvent(s) into hydrocarbons.

According to an embodiment of the present invention, the weight ratio H₂S:scavenging composition ranges from 1:2 to 1:0.05, preferably from 1:1.5 to 1:0.1, more preferably from 1:1 to 1:0.3, even more preferably from 1:0.8 to 1:0.4 and advantageously from 1:0.8 to 1:0.4. In this ratio, H₂S represents the amount of hydrogen sulphide in the hydrocarbons, before contacting with the scavenging composition of the invention.

Hydrocarbons can be selected from natural gas, liquefied petroleum gas (LPG), finished fuels such as diesel, kerosene or gasoline, crude and heavy residual oils, and asphalt.

Hydrocarbons contain H₂S and/or mercaptans, in an amount for example ranging from 1 to 10 000 ppm. Mercaptans that can be removed from hydrocarbon streams within the framework of the present invention may be C₁-C₆ mercaptans, such as C₁-C₄ mercaptans. The scavenging composition of the invention may represent from 0.0005 to 5 % by weight of the total weight of the hydrocarbon-containing composition.

The scavenging compositions according to the invention can be used in LPG when contaminated with H₂S, e.g. after a unit upset. Presence of H₂S in LPG may cause metal corrosion and a potential health hazard to consumers. Water-soluble scavengers are generally recommended for LPG because they will separate completely from the hydrocarbon and prevent contamination of the LPG with materials of lower volatility. The presence of lower-volatility components in LPG is undesirable because these materials do not burn as well and could cause injector plugging and fouling on burner tips.

The scavenging compositions according to the invention can be used in finished fuels such as gasoline, kerosene and diesel which are required to be noncorrosive. Oil-soluble, nonreversible H₂S scavengers are typically the product of choice because they will not adversely affect critical fuel properties and will not add water (and possibly a haze) to a finished fuel.

The scavenging compositions according to the invention can be used in crude and heavy residual oils contain significant concentrations of H₂S as a natural component and/or as a result of thermal cracking processes that break apart high molecular weight sulfur-containing compounds to generate H₂S.

The scavenging compositions according to the invention can be used in asphalt, which contains extremely high levels of H₂S, often exceeding 1% (10,000 ppm). Asphalt is the heaviest of the products coming out of the refinery and typically the product in which sulfur compounds concentrate. Because of the high viscosity of asphalt, it must be stored at high temperatures (300 to 400°F). These temperatures promote cracking of sulfur-containing compounds and formation of H₂S. Moreover, asphalt has a high vapor:liquid partition coefficient (400:1), meaning that H₂S tends to collect in the vapor phase. The combination of high temperatures, high H₂S concentrations, and high viscosity makes asphalt challenging to treat. It is especially critical to lower the H₂S content because asphalt is shipped by rail car and tank truck, and exposure of personnel and consumers is a real concern. Because of the elevated temperatures of asphalt applications, water-soluble scavengers generally are not suitable; rather, oil-soluble carriers for scavengers are preferred.

The scavenging compositions according to the invention may also be used in drilling fluids, in oil-based drilling fluids or in water-based drilling fluids, for drilling applications.

The present invention is also directed to a drilling fluid comprising the scavenging composition of the invention, preferably in an amount of at least 10 ppm by weight, based on the total weight of the scavenging composition.

### Water-containing composition

The invention is also directed to a water-containing composition comprising:
- at least 70%wt of water, and
- at least 10 ppm by weight of the compound(s) as defined in the invention, preferably at least 1%wt, more preferably at least 10%wt of the compound(s) as defined in the invention,
- optionally at least 10 ppm by weight of one or more solvent(s) different from water.

Preferably, the compound comprises one or more of the features defined above in relation to the composition of the invention.

Preferably, the solvent comprises one or more of the features defined above in relation to the composition of the invention.

The water-containing composition can be prepared by adding the scavenging composition of the invention comprising the compound of the invention alone or in the presence of solvent(s) into a water-containing media. The water-containing media is preferably a water-based mud.

According to an embodiment, the water-containing composition of the invention is a water-based mud comprising the scavenging composition of the invention.

According to an embodiment of the present invention, the weight ratio H₂S:scavenging composition ranges from 1:2 to 1:0.05, preferably from 1:1.5 to 1:0.1, more preferably from 1:1 to 1:0.3, even more preferably from 1:0.8 to 1:0.4 and advantageously from 1:0.8 to 1:0.4. In this ratio, H₂S represents the amount of hydrogen sulphide in the hydrocarbons, before contacting with the scavenging composition of the invention.

The invention is thus particularly useful in order to reduce hydrogen sulphide amount of water-based drilling fluids.

### EXAMPLES

The invention is now described with the help of the following examples, which are not intended to limit the scope of the present invention but are incorporated to illustrate advantages of the present invention and best mode to perform it. The following examples also demonstrate the synthesis of different compounds of the invention and the effectiveness of H₂S scavengers of the invention.
The compounds of the invention can be characterized by IR, ¹H NMR and ¹³C NMR spectroscopic techniques.

### EXAMPLE 1: General synthesis of the compounds of the invention

Compounds detailed in table 1 above have been prepared.
Table 2 below details the primary amines and the mole ratio of BMOM and primary amines involved:

**Table 2: details of the compounds prepared**

| | Primary amines | Molar ratio BMOM/primary amines |
|---|---|---|
| I-A | Monoethanolamine (MEA) | 1/1 |
| I-B | Aminoethylethanolamine (AEEA) | 1/1 |
| I-C | Benzylamine (BA) | 1/1 |
| I-D | N,N-dimethylethylenediamine (DEDA) | 1/1 |
| I-E | n-butylamine (NBA) | 1/1 |
| I-F | N,N-dimethylpropane-1,3-diamine (DMAPA) | 1/1 |
| I-G | 1,2-ethylenediamine (EDA) | 1/1 |
| I-H | 1,3-propyleneamine (PDA) | 1/1 |
| I-I | Diethylenetriamine (DETA) | 1/1 |
| I-J | 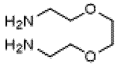 Jeffamine^{®} EDR-148 (EDR-148) | 1/1 |
| I-K | 4-(2-Aminoethyl)morpholine (AEM) | 1/1 |
| I-L | 2-Ethyl-1-hexylamine (2EHA) | 1/1 |
| I-M | 1-Dodecylamine (DDA) | 1/1 |
| I-N | 1,2-ethylenediamine (EDA) | 2/1 |
| I-O | 1,3-propanediamine (PDA) | 2/1 |
| I-P | Diethylenetriamine (DETA) | 2/1 |
| I-Q | 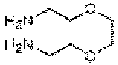 Jeffamine^{®} EDR-148 (EDR-148) | 2/1 |
| I-R | p-Xylylenediamine (XLDA) | 2/1 |
| I-S | Tris(20aminoethyl)amine (TAEA) | 3/1 |

Compound I-E (1,1'-(5-butyl-1,3,5-triazinane-1,3-Diyl)bis(propan-2-ol) has been prepared according to the following process:
A three neck round bottom flask equipped with reflux condenser, dropping funnel and a thermometer, was charged with 186.26 gm (1 mole) of bis-(5-methyloxazolidine-3-yl)methane, it was heated to 40°C. 73.14 (1 mole) of 1-butylamine was then added dropwise over a period of 30 min under mechanical stirring. Resultant mixture was heated at 60°C for 6 hours. A colorless viscous reaction product was obtained with theoretical yields and with 99% purity. Product was well characterized by 1H, 13C, DEPT135 NMR and Mass spectroscopy. Residual formaldehyde was also analyzed using GC-head space techniques, which was found to be 157.851 ppm.

The spectral data of the product (compound I-E) are the following:
**IR:** - 3361.70, 3298.26, 2959.74, 2928.49, 2864.17, 2820.81, 1672.64, 1597.65 and 1456.37 cm⁻¹
**1H NMR:** 0.846 - 0.882, t, 3H, 1x- >N-CH₂-CH₂-CH₂-CH₃; 0.971 - 1.02, t, 6H, 2x-CH₃ ; 1.24 - 1.37, m, 4H, >N-CH₂-CH₂-CH₂-CH₃ ; 2.16 - 2.38, m, 6H, 2x >N-CH₂CH(OH)CH₃& >N-CH₂-CH₂-CH₂-CH₃ ; 3. 22-3. 60, m, 6H, 3x ->N-CH₂-N<; 3.72 - 3.78, q, 2H, 2x >N-CH₂CH(OH)CH₃; 4.70, broad, 2H, -OH.
**13C NMR:** 13.85, 1x- >N-CH₂-CH₂-CH₂-CH₃; 20.15, -N-CH₂-CH₂-CH₂-CH₃; 21.47, 2x-CH₃; 29.28, ->N-CH₂-CH₂-CH₂-CH₃; 51.55, >N-CH₂-CH₂-CH₂CH₃ ; 61.05 & 61.72, 2x >N-CH₂-CH(OH)CH₃ ; 64.13, >N-CH₂-CH(OH)CH₃; 74.58 & 74.65, 3x >N-CH₂-N<.
**DEPT135:** Peaks at 13.85, 21.47 and 64.11 are +ve peaks. Whereas, peaks at 20.15, 28.98, 51.53, 61.05 & 61.72, 74.57 & 74.64 are -ve peaks.

Other compounds I-A to I-D and I-F to I-M have been prepared with a similar process as compound I-E but with a different primary amine, as detailed in Table 2.

Compound I-O (1,1',1",1‴-(propane-1,3-diylbis(1,3,5-triazinane-5,1,3-triyl))tetrakis(propan-2-ol) has been prepared according to the following process:
A three neck round bottom flask equipped with reflux condenser, dropping funnel and a thermometer, was charged with 352.52 gm (2 moles) of bis-(5-methyloxazolidine-3-yl)methane, it was heated to 40°C. 74.13 gm (1 mole) of 1,3-propanediamine was then added dropwise over a period of 30 min under mechanical stirring. Resultant mixture was heated at 60°C for 6 hours. A pale-yellow viscous reaction product was obtained with theoretical yields and with 99% purity. Product was well characterized by 1H, 13C, DEPT135 NMR and Mass spectroscopy. Residual formaldehyde was also analyzed using GC-head space techniques, which was found to be 176.026 ppm.

The spectral data of the product (compound I-O) are the following:
**IR:** 3374.40, 2963.55, 2857.17, 2807.44, 1667.94, 1406.09 cm⁻¹.
**1H NMR:** 1.007 & 1.027, 2s, 12H, 4x -CH₃; 1.494, m, 2H, >N-CH₂-CH₂-CH₂-N<; 2.285 - 2.426, d, 4H, >N-CH₂-CH₂-CH₂-N<; 3.033 - 3.503, m, 12H, 6x >N-CH₂-N<; 3.709-3.770, q, 4H, 4x >N-CH₂-CH(CH₃)-OH; 4.617, broad, 4H, 4x >N-CH₂-CH(CH₃)-OH.
**13C NMR:** 21.636, 4x -CH₃; 23.227, >N-CH₂-CH₂-CH₂-N<; 51.621, >N-CH₂-CH₂-CH₂-N<; 60.619, 4x >N-CH₂-CH(CH₃)-OH; 64.114, 4x >N-CH₂-CH(CH₃)-OH; 74.679, 6x >N-CH₂-N<.
**DEPT:** Peaks at 22.066, 64.554 are +ve peaks, and peaks at 23.655, 52.051, 61.028, 75.109 are -ve peaks.

Other compounds I-N, I-P to I-R have been prepared with a similar process as compound I-O but with a different primary amine, as detailed in Table 2.

Compound I-S (1,1',1",1‴,1ʺʺ,1‴ʺ-((nitrilotris(ethane-2,1-diyl))tris(1,3,5-triazinane-5,1,3-triyl))hexakis(propan-2-ol) has been prepared according to the following process:
A three neck round bottom flask equipped with reflux condenser, dropping funnel and a thermometer, was charged with 558.78 gms (3 moles) of bis-(5-methyloxazolidine-3-yl)methane, it was heated to 40°C. 146.24 (1 mole) of tris(2-aminoethyl)amine was then added dropwise over a period of 60 min under mechanical stirring. Resultant mixture was heated at 80°C for 6 hours. An orange colored viscous reaction product was obtained with theoretical yields and with 99% purity. Product was well characterized by 1H, 13C, DEPT135 NMR and Mass spectroscopy. Residual formaldehyde was also analyzed using GC-head space techniques, which was found to be 180.278 ppm.

The spectral data of the product (compound I-S) are the following:
**IR:** 3287.63, 3189.71, 2961.64, 2925.70, 2863.18, 1563.96 and 1454.81 cm⁻¹.
**1H NMR:** 0.979, d, 18H, 6x -CH₃; 2.372 - 2.540, m, 2H, 2x >N-CH₂-C; 3.21, m, 18H, 3x >N-CH₂-N<; 3.766, m, 3H, 3x >N-CH₂-CH(CH₃)-OH and, 4.723, broad, 6H, 6x >N-CH₂-CH(CH₃)-OH.
**13C NMR:** 20.89, 6x -CH₃; 48.58 & 49.69, 6C, 3x >N-CH₂-CH₂-N<; 60.541 & 60.779, 6x >N-CH₂-CH(CH₃)-OH; 64.096, 6x >N-CH₂-CH(OH)CH₃; 74.64, 9x >N-CH₂-N<.
**DEPT:** Peaks at 20.88 and 64.09 are +ve peaks, and, 48.57, 49.69, 60.53, 60.76 and 74.64 are -ve peaks.

### EXAMPLE 2: Measurement of H₂S scavenging ability of the scavenging compositions

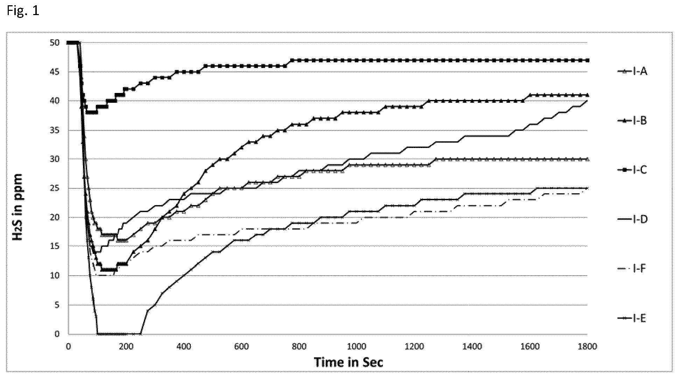

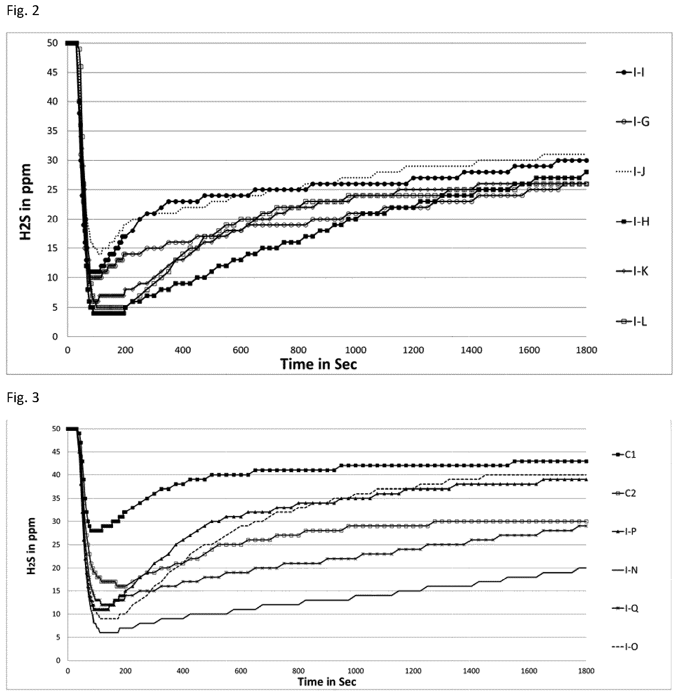

Scavenging compositions have been prepared by mixing 50%wt of one scavenger compound and 50%wt of butyl carbitol. Scavenger compounds used in the present example are:
- comparative compounds: MEA-triazine (named C1 in Fig. 3) and MBO (named C2 in Fig. 3)
- compounds detailed in example 1 (compounds I-A, I-B, I-C, I-D, I-E, I-F in Fig. 1 ; compounds I-G, I-H, I-I, I-J, I-K, I-L in Fig. 2 and compounds I-N, I-O, I-P, I-Q in Fig. 3).

In this example, the kinetic of the H2S scavenging effect of the scavenging compositions had been evaluated.

In a typical experiment, dry H₂S gas with 50 ppm of concentration being passed through the hydrocarbon media, with 0.3L/min flow rate and 1.5 psi pressure (at 25°C with a stirring speed of 1000 rpm). The H₂S scavenger being dosed into gas purging bottle of 250 mL, gas purging bottle of 250 mL, was charged with 100 mL hydrocarbon media. H2S gas with 50 ppm concentration was passed through this and concentration of H2S at the outlet of this gas purging bottle was monitored with H2S. Once the concentration of H2S at the outlet being observed as 50 ppm, H2S scavenger, typically 0.4 ml of scavenger dissolved in 1.6 mL of butyl Carbitol was charged using syringe. The outlet of this gas purging bottle is passed through the gas scrubber. The gas scrubber typically scrubs all the gases and vapor apart from H₂S and allows to pass only H₂S gas to the detector (e.g. an electrochemical sensor). The detector shows the actual concentration of H₂S in real time throughout the experiment. Finally, the outlet from the H₂S detector is passed through aq. NaOH solution in order to neutralize the H₂S gas.

The test had been performed in a dearomatized hydrocarbon solvent (HC1) having an initial boiling point higher than 120°C, a final boiling point higher than 250°C and a flash point above 100°C with aromatic content less than 0.05%wt has been used for the tests.

The following protocol had been implemented:
1. Transfer 100 mL of hydrocarbon solvent (HC1 detailed above) to gas-purging bottle. Fit the fritted glass bubbler at the opening of the bottle. Assemble the gas purging bottle/glass frit apparatus. Place the bottle into the oil bath.
2. Fit one end of the Tygon tube at the outlet of the H₂S gas cylinder, and fit another end to a gas flowmeter. Connect the outlet of the gas flowmeter to inlet of the fritted glass bubbler using Tygon tube. Outlet of the condenser will be connected to gas scrubber, to ensure all the other gases get trapped and only H₂S goes to the detector.
3. Finally, outlet of the detector is attached to H₂S neutralizing unit which contains aq. NaOH solution.
4. Start the flow of H₂S gas with 50 ppm concentration in N₂. Ensure that the pressure maintain to 0.3 L/min all the times, with 1.5 psi pressure.
5. Purge the content of the gas purging bottle for at least 15-20 min, to remove all the dissolved oxygen, and till constant 50 ppm reading is shown at H₂S detector.
6. Once the constant 50 ppm concentration is displayed at H₂S detector, measured quantity of the H₂S scavenger to be injected using syringe through the H₂S scavenger dosing point into the hydrocarbon media present in the gas purging bottle.
7. H₂S detector is inbuild with data logger which save the readings after every 5 seconds delay.
8. Upon completion of the experiment, typically being run for 30 min. The H₂S detector is connected to computer to retrieve the data.

The H₂S amount in ppm in function of the time had been measured and is shown in Fig. 1, Fig. 2 and Fig. 3.

The results illustrated in Fig. 1 to in Fig. 3 show that the scavenging compositions of the invention (comprising compounds of the invention detailed in Table 1) provide very satisfying scavenging performances, in particular much improved scavenging performances than comparative composition C1 comprising MEA-triazine.

## Claims

1. A compound of formula (I): 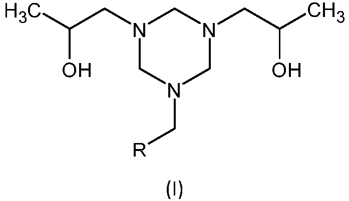 wherein
- R is a radical selected from a linear, branched or cyclic hydrocarbyl group optionally comprising one or more heteroatoms, such as oxygen, sulfur, halogen, silicon, nitrogen atoms.

2. Compound according to claim 1, wherein R is selected from: R¹-OH, R¹-[O-R²]ₚ-OH, optionally substituted benzyl, R¹-NR³R⁴, R⁵, R¹-[N(H)-R²]ₙ-NR³R⁴, R¹-[O-R²]ₚ-NR³R⁴, R¹-Q, R¹-[N(H)-R²]ₙ-Q, R¹-[OR²]ₚ-Q, R¹-N(R³-Q)(R⁴-Q) wherein:
- R¹ is selected from a linear, branched or cyclic alkyl or alkenyl radical having from 1 to 24 carbon atoms, preferably from 1 to 16 carbon atoms, more preferably from 1 to 12 carbon atoms, even more preferably from 1 to 8 carbon atoms or from an aromatic radical optionally substituted by one or more substituent selected from C₁-C₆-alkyl, hydroxyl, C₁-C₆-alkoxy, or C₁-C₆-alkylamine, preferably a phenyl radical substituted by C₁-C₆-alkyl;
- R² is a linear, branched or cyclic alkyl or alkenyl radical having from 1 to 24 carbon atoms, preferably from 1 to 16 carbon atoms, more preferably from 1 to 12 carbon atoms, even more preferably from 1 to 8 carbon atoms;
- R³ and R⁴ are, independently to each other, a hydrogen atom or a linear, branched or cyclic alkyl or alkenyl radical having from 1 to 16 carbon atoms, preferably from 1 to 12 carbon atoms, more preferably from 1 to 8 carbon atoms, even more preferably from 1 to 4 carbon atoms;
- R⁵ is a linear, branched or cyclic alkyl or alkenyl radical having from 1 to 30 carbon atoms, preferably from 1 to 24 carbon atoms, more preferably from 1 to 16 carbon atoms, even more preferably from 1 to 12 carbon atoms;
- Q is a radical selected from morpholine, piperidine, piperazine, or the monovalent radical of formula: 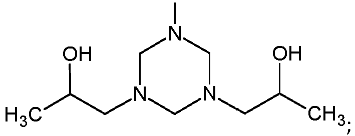
- the optionally substituted benzyl can be a benzyl substituted by one or more substituent selected from C₁-C₆-alkyl, hydroxyl, C₁-C₆-alkoxy, C₁-C₆-alkylamine;
- n is an integer ranging from 1 to 10, preferably from 1 to 6, more preferably from 1 to 4, even more preferably from 1 to 2;
- p is an integer ranging from 1 to 10, preferably from 1 to 6, more preferably from 1 to 4, even more preferably from 1 to 2.

3. Compound according to claim 1 or 2, wherein R is selected from: R¹-OH, R¹-O-R²-OH, benzyl, R¹-NR³R⁴, R⁵, R¹-N(H)-R²-NR³R⁴, R¹-O-R²-O-R²-NR³R⁴, R¹-Q, R¹-N(H)-R²-Q, R¹-O-R²-O-R²-Q, R¹-N(R³-Q)₂ wherein:
- R¹ is selected from a linear or branched alkyl radical having from 1 to 16 carbon atoms, preferably from 1 to 12 carbon atoms, more preferably from 1 to 8 carbon atoms, even more preferably from 1 to 4 carbon atoms or from a phenyl radical substituted by C₁-C₆-alkyl;
- R² is a linear or branched alkyl radical having from 1 to 16 carbon atoms, preferably from 1 to 12 carbon atoms, more preferably from 1 to 8 carbon atoms, even more preferably from 1 to 4 carbon atoms;
- R³ and R⁴ are, independently to each other, a hydrogen atom or a linear or branched alkyl radical having from 1 to 16 carbon atoms, preferably from 1 to 12 carbon atoms, more preferably from 1 to 8 carbon atoms, even more preferably from 1 to 4 carbon atoms;
- R⁵ is a linear or branched alkyl radical having from 1 to 24 carbon atoms, preferably from 1 to 16 carbon atoms, more preferably from 1 to 12 carbon atoms, even more preferably from 1 to 8 carbon atoms;
- Q is a morpholine radical or the monovalent radical of formula:

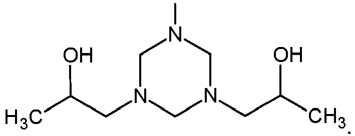

4. Compound according to any one of claims 1 to 3, wherein R is selected from: R¹-OH, R¹-O-R²-OH, benzyl, R¹-NR³R⁴, R⁵, R¹-N(H)-R²-NR³R⁴, R¹-O-R²-O-R²-NR³R⁴, R¹-Q, R¹-N(H)-R²-Q, R¹-O-R²-O-R²-Q, R¹-N(R³-Q)₂ wherein:
- R¹ is a linear alkyl radical having from 1 to 4 carbon atoms;
- R² is a linear alkyl radical having from 1 to 4 carbon atoms;
- R³ and R⁴ are, independently to each other, a hydrogen atom or a linear alkyl radical having from 1 to 4 carbon atoms;
- R⁵ is a linear alkyl radical having from 1 to 8 carbon atoms;
- Q is a morpholine radical or the monovalent radical:

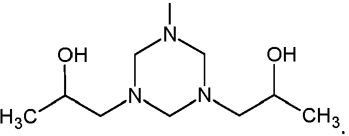

5. Compound according to any one of claims 1 to 4, replying to one of the following formula:

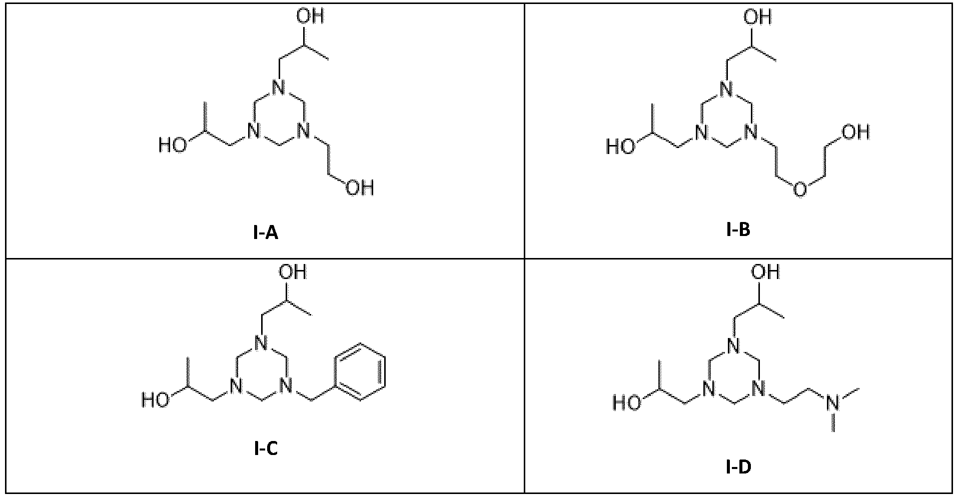

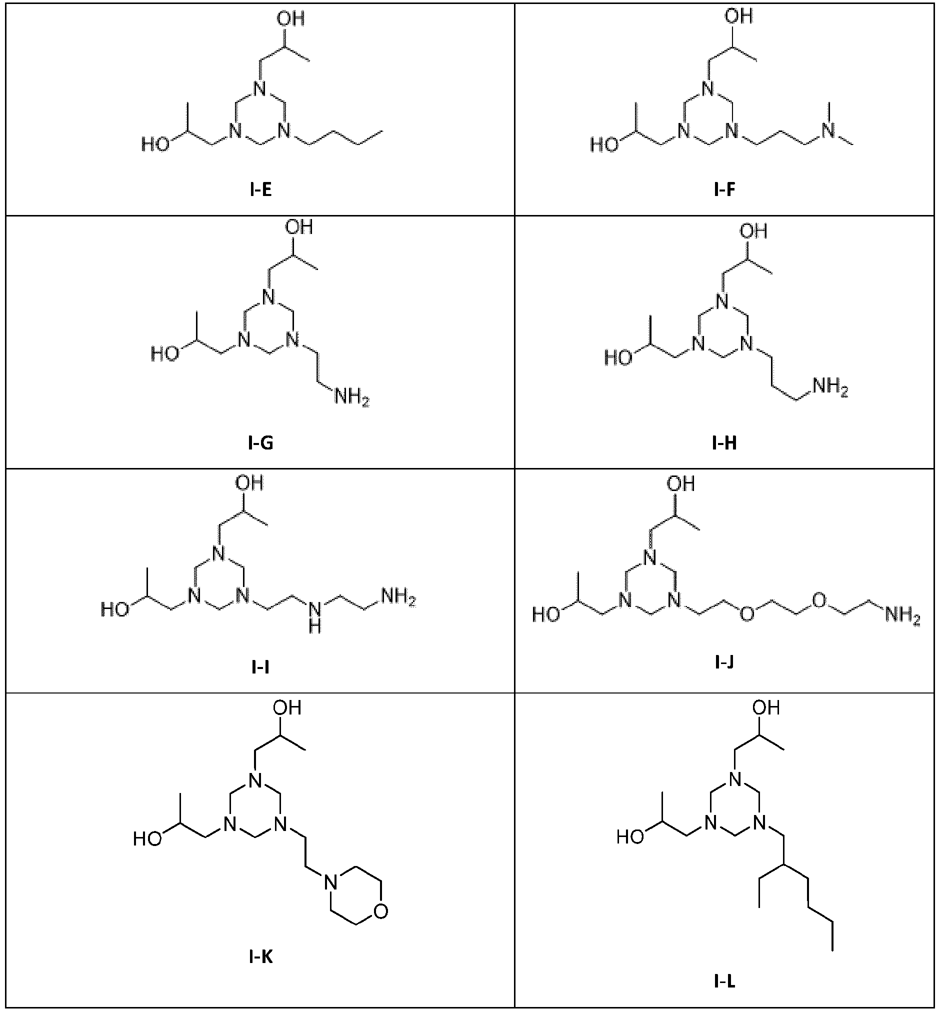

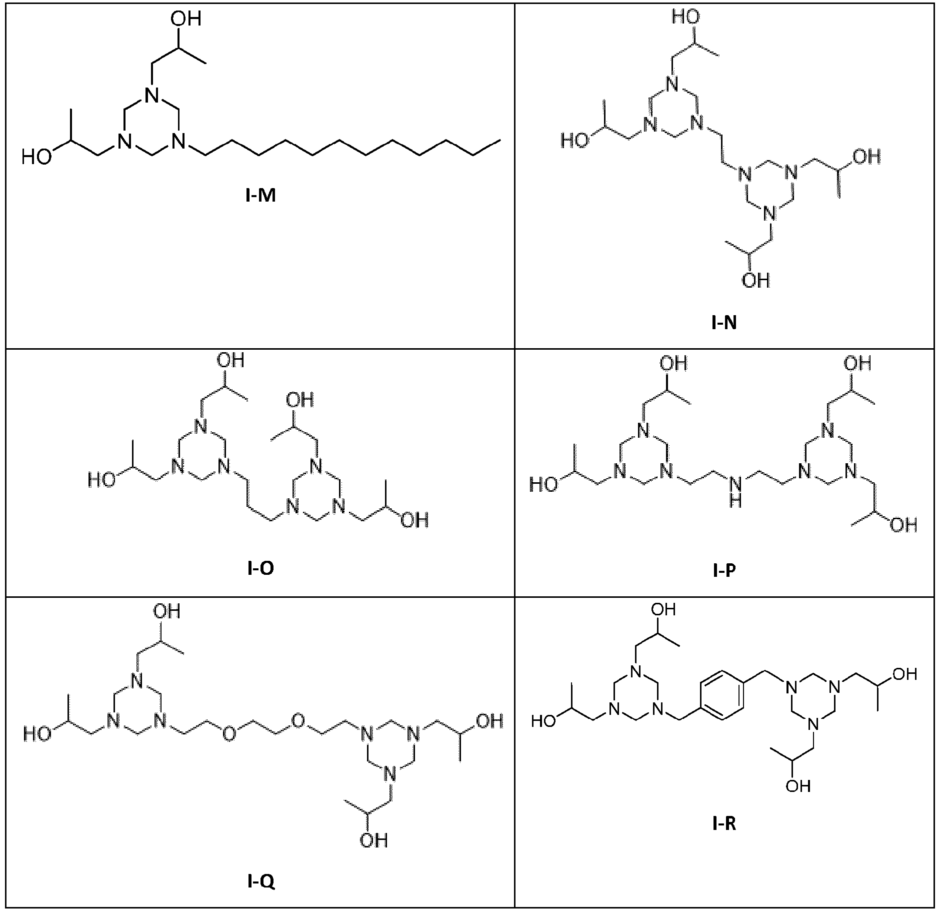

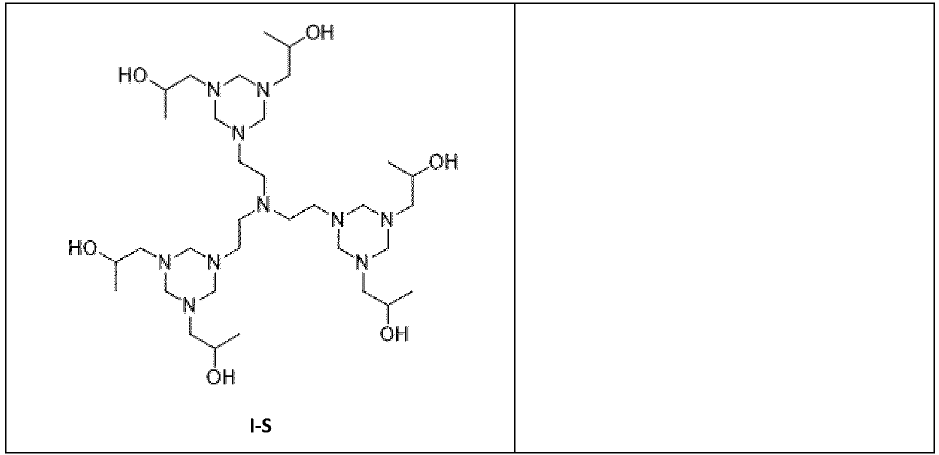

6. Process for manufacturing the compound according to any one of claims 1 to 5, comprising a step of contacting at least one bis(5-methyloxazolidin-3-yl)methane (BMOM) with at least one primary amine.

7. Process according to claim 6, wherein the contacting step is performed with a molar ratio BMOM:primary amines ranging from 1:5 to 5:1, preferably from 1:1 to 3:1.

8. Composition comprising:
- from 20 to 98%wt of at least one compound according to any one of claims 1 to 5, and
- from 2 to 80%wt of solvent(s),
based on the total weight of the composition.

9. Use of the compound according to any one of claims 1 to 5 or of the composition according to claim 8, to scavenge hydrogen sulphide and mercaptans in streams selected from hydrocarbon streams and water-containing streams.

10. Use according to claim 9, wherein the compound is added in an amount of at least 1%wt, based on the total weight of hydrogen sulphide and mercaptans in the stream.

11. Use according to claim 9 or 10, wherein the streams are selected from hydrocarbon-containing streams such as liquefied petroleum gas (LPG), finished fuels, crude and heavy residual oils, natural gas asphalt, oil-based drilling fluids, and water-containing streams such as water-based drilling fluids.

12. A hydrocarbon-containing composition comprising hydrocarbons and the compound according to any one of claims 1 to 5 or of the composition according to claim 8.

13. A water-containing stream comprising water and the compound according to any one of claims 1 to 5 or of the composition according to claim 8.

## Patentansprüche

1. Verbindung von Formel (I): 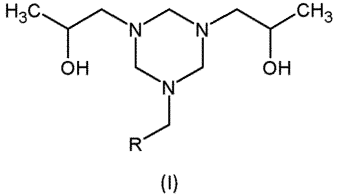 wobei
R ein Radikal ist, ausgewählt aus einer linearen, verzweigten oder cyclischen Kohlenwasserstoffgruppe, optional umfassend ein oder mehrere Heteroatome, wie beispielsweise Sauerstoff-, Schwefel-, Halogen-, Silizium- oder Stickstoffatome.

2. Verbindung nach Anspruch 1, wobei R ausgewählt ist aus: R¹-OH, R¹-[O-R²]ₚ-OH, optional substituiertem Benzyl, R¹-NR³R⁴, R⁵, R¹-[N(H)-R²]ₙ-NR³R⁴, R¹-[O-R²]ₚ-NR³R⁴, R¹-Q, R¹-[N(H)-R²]ₙ-Q, R¹-[O-R²]ₚ-Q, R¹-N(R³-Q)(R⁴-Q), wobei:
- R¹ ausgewählt ist aus einem linearen, verzweigten oder cyclischen Alkyl- oder Alkenylradikal, das 1 bis 24 Kohlenstoffatome, vorzugsweise 1 bis 16 Kohlenstoffatome, bevorzugter 1 bis 12 Kohlenstoffatome, noch bevorzugter 1 bis 8 Kohlenstoffatome aufweist, oder aus einem aromatischen Radikal, optional substituiert durch einen oder mehrere Substituenten, ausgewählt ausC₁-C₆-Alkyl, Hydroxyl, C₁-C₆-Alkoxy oder C₁-C₆-Alkylamin, vorzugsweise einem Phenylradikal, substituiert durch C₁-C₆-Alkyl;
- R² ein lineares, verzweigtes oder cyclisches Alkyl- oder Alkenylradikal ist, das 1 bis 24 Kohlenstoffatome, vorzugsweise 1 bis 16 Kohlenstoffatome, bevorzugter 1 bis 12 Kohlenstoffatome, noch bevorzugter 1 bis 8 Kohlenstoffatome aufweist;
- R³ and R⁴ unabhängig voneinander ein Wasserstoffatom oder ein lineares, verzweigtes oder cyclisches Alkyl- oder Alkenylradikal ist, das 1 bis 16 Kohlenstoffatome, vorzugsweise 1 bis 12 Kohlenstoffatome, bevorzugter 1 bis 8 Kohlenstoffatome, noch bevorzugter 1 bis 4 Kohlenstoffatome aufweist;
- R⁵ ein lineares, verzweigtes oder cyclisches Alkyl- oder Alkenylradikal ist, das 1 bis 30 Kohlenstoffatome, vorzugsweise 1 bis 24 Kohlenstoffatome, bevorzugter 1 bis 16 Kohlenstoffatome, noch bevorzugter 1 bis 12 Kohlenstoffatome aufweist;
- Q ein Radikal ist, ausgewählt aus Morpholin, Piperidin, Piperazin oder dem einwertigen Radikal folgender Formel: 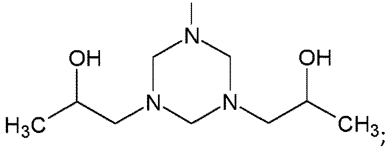
- das optional substituierte Benzyl ein Benzyl sein kann, substituiert durch einen oder mehrere Substituenten, ausgewählt aus C₁-C₆-Alkyl, Hydroxyl, C₁-C₆-Alkoxy, C₁-C₆-Alkylamin;
- n eine ganze Zahl in einem Bereich von 1 bis 10, vorzugsweise von 1 bis 6, bevorzugter von 1 bis 4, noch bevorzugter von 1 bis 2 ist;
- p eine ganze Zahl in einem Bereich von 1 bis 10, vorzugsweise von 1 bis 6, bevorzugter von 1 bis 4 und noch bevorzugter von 1 bis 2 ist.

3. Verbindung nach Anspruch 1 oder 2, wobei R ausgewählt ist aus: R¹-OH, R¹-O-R²-OH, Benzyl, R¹-NR³R⁴, R⁵, R¹-N(H)-R²-NR³R⁴, R¹-O-R²-O-R²-NR³R⁴, R¹-Q, R¹-N(H)-R²-Q, R¹-O-R²-O-R²-Q, R¹-N(R³-Q)₂
wobei:
- R¹ ausgewählt ist aus einem linearen oder verzweigten Alkylradikal, das 1 bis 16 Kohlenstoffatome, vorzugsweise 1 bis 12 Kohlenstoffatome, bevorzugter 1 bis 8 Kohlenstoffatome, noch bevorzugter 1 bis 4 Kohlenstoffatome aufweist, oder aus einem Phenylradikal, substituiert durch C₁-C₆-Alkyl;
- R² ein lineares oder verzweigtes Alkylradikal ist, das 1 bis 16 Kohlenstoffatome, vorzugsweise 1 bis 12 Kohlenstoffatome, bevorzugter 1 bis 8 Kohlenstoffatome, noch bevorzugter 1 bis 4 Kohlenstoffatome aufweist;
- R³ and R⁴ unabhängig voneinander ein Wasserstoffatom oder ein lineares oder verzweigtes Alkylradikal ist, das 1 bis 16 Kohlenstoffatome, vorzugsweise 1 bis 12 Kohlenstoffatome, bevorzugter 1 bis 8 Kohlenstoffatome, noch bevorzugter 1 bis 4 Kohlenstoffatome aufweist;
- R⁵ ein lineares oder verzweigtes Alkylradikal ist, das 1 bis 24 Kohlenstoffatome, vorzugsweise 1 bis 16 Kohlenstoffatome, bevorzugter 1 bis 12 Kohlenstoffatome, noch bevorzugter 1 bis 8 Kohlenstoffatome aufweist;
- Q ein Morpholinradikal oder das einwertige Radikal folgender Formel ist:

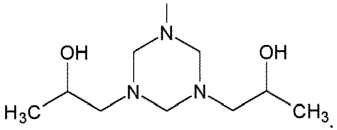

4. Verbindung nach einem der Ansprüche 1 bis 3, wobei R ausgewählt ist aus: R¹-OH, R¹-O-R²-OH, Benzyl, R¹-NR³R⁴, R⁵, R¹-N(H)-R²-NR³R⁴, R¹-O-R²-O-R²-NR³R⁴, R¹-Q, R¹-N(H)-R²-Q, R¹-O-R²-O-R²-Q, R¹-N(R³-Q)₂, wobei:
- R¹ ein lineares Alkylradikal ist, das 1 bis 4 Kohlenstoffatome aufweist;
- R² ein lineares Alkylradikal ist, das 1 bis 4 Kohlenstoffatome aufweist;
- R³ und R⁴ unabhängig voneinander ein Wasserstoffatom oder ein lineares Alkylradikal, das 1 bis 4 Kohlenstoffatome aufweist, sind;
- R⁵ ein lineares Alkylradikal ist, das 1 bis 8 Kohlenstoffatome aufweist;
- Q ein Morpholinradikal oder das einwertige Radikal ist:

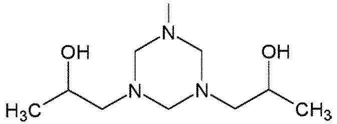

5. Verbindung nach einem der Ansprüche 1 bis 4, die einer der folgenden Formeln entspricht:

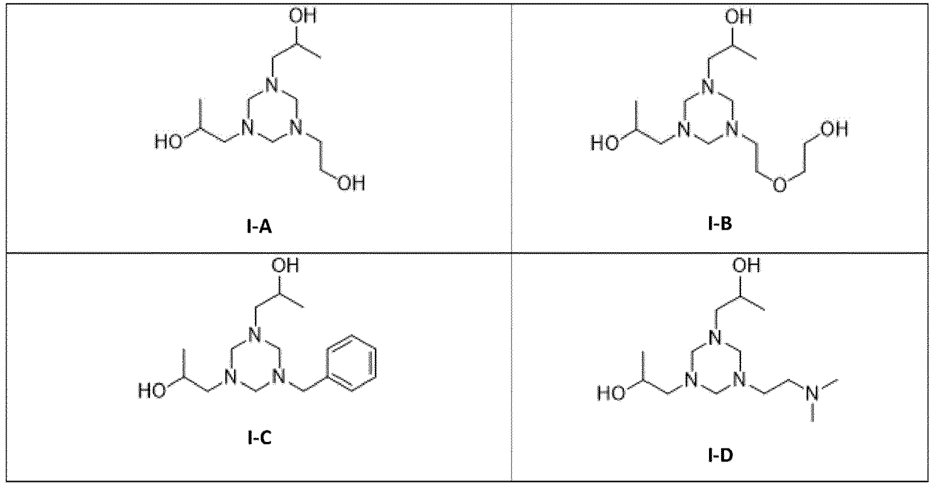

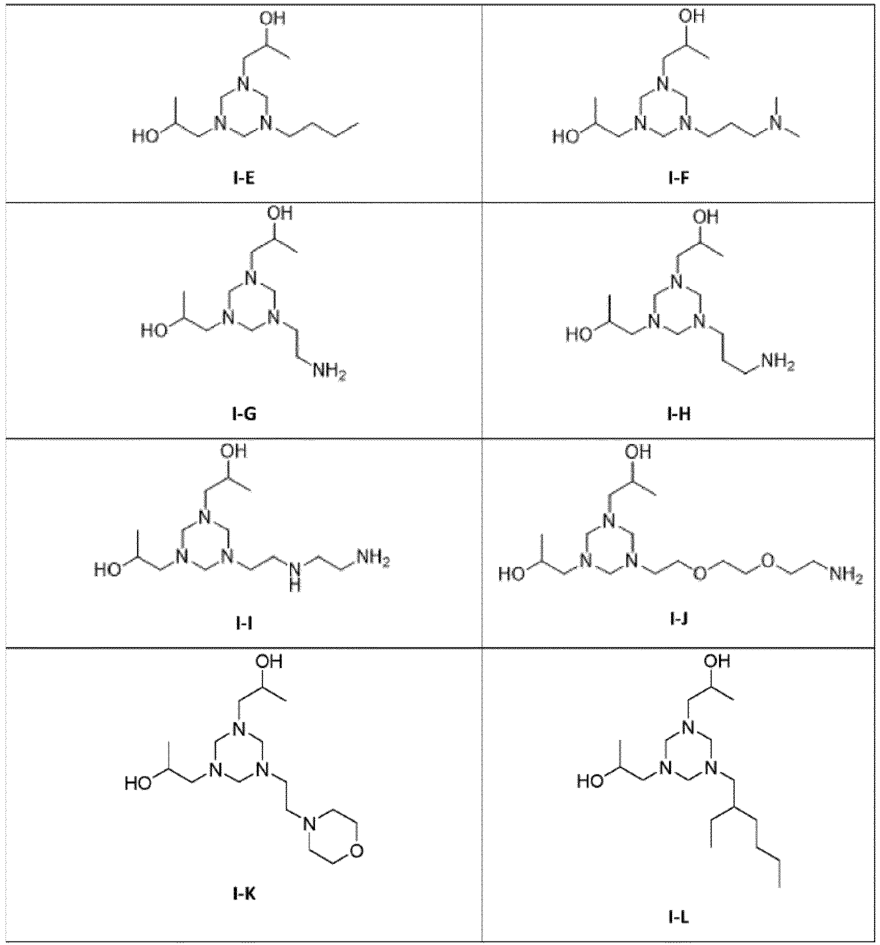

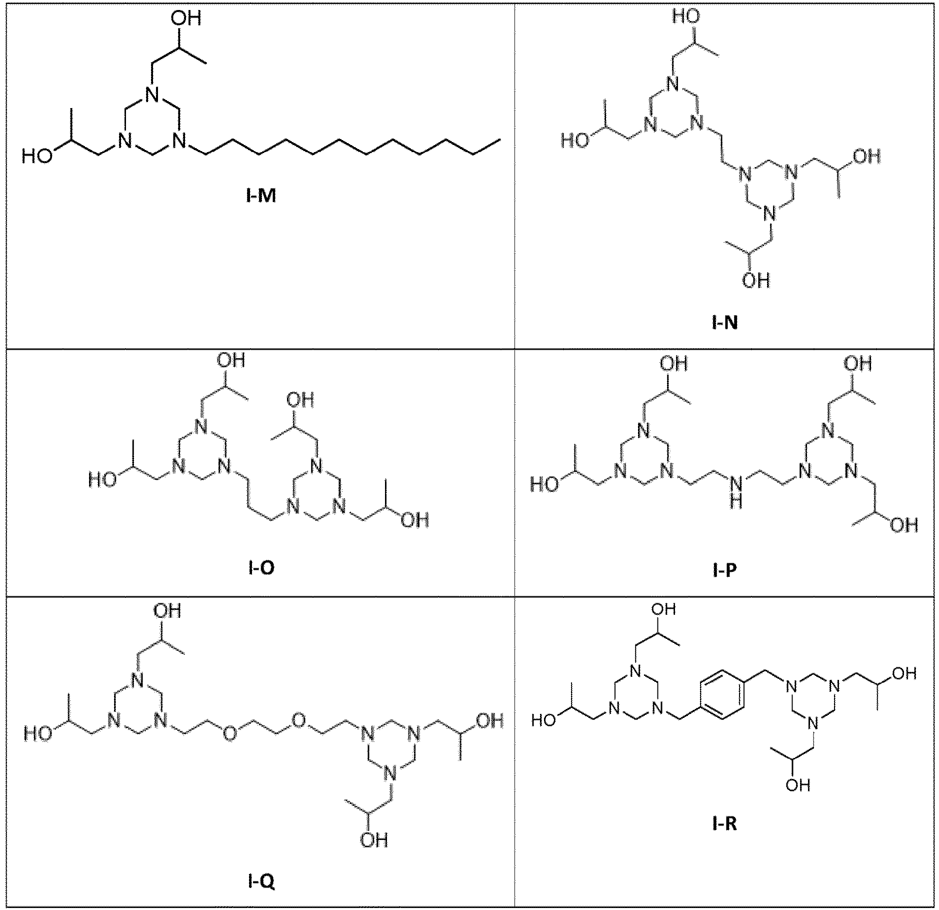

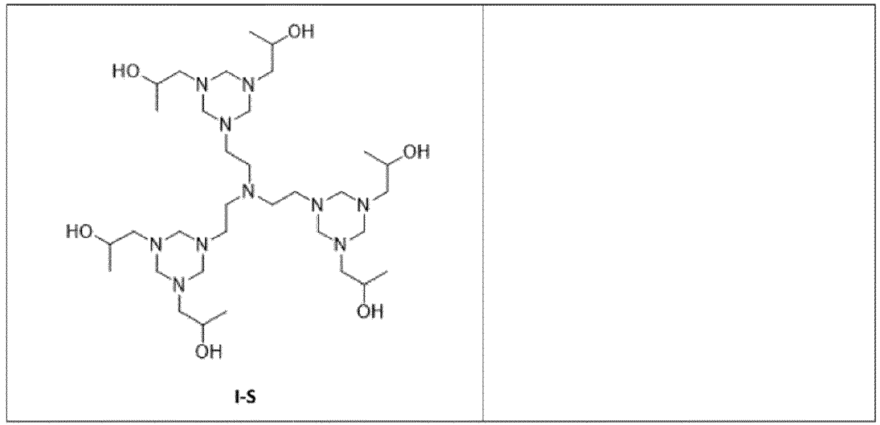

6. Verfahren zum Herstellen der Verbindung nach einem der Ansprüche 1 bis 5, umfassend einen Schritt eines Inkontaktbringens mindestens eines bis(5-Methyloxazolidin-3-yl)methans (BMOM) mit mindestens einem Primäramin.

7. Verfahren nach Anspruch 6, wobei der Schritt eines Inkontaktbringens mit einem molaren Verhältnis BMOM:Primäramine in einem Bereich von 1:5 bis 5:1, vorzugsweise von 1:1 bis 3:1, ausgeführt wird.

8. Zusammensetzung, umfassend:
- 20 bis 98 Gewichts-% mindestens einer Verbindung nach einem der Ansprüche 1 bis 5, und
- 2 bis 80 Gewichts-% von Lösungsmittel(n),
bezogen auf das Gesamtgewicht der Zusammensetzung.

9. Verwendung der Verbindung nach einem der Ansprüche 1 bis 5 oder der Zusammensetzung nach Anspruch 8, um Schwefelwasserstoff und Mercaptane in Strömen, ausgewählt aus Kohlenwasserstoffströmen und wasserhaltigen Strömen, einzufangen.

10. Verwendung nach Anspruch 9, wobei die Verbindung in einer Menge von mindestens 1 Gewichts-%, bezogen auf das Gesamtgewicht von Schwefelwasserstoff und Mercaptanen in dem Strom, hinzugefügt wird.

11. Verwendung nach Anspruch 9 oder 10, wobei die Ströme ausgewählt sind aus kohlenwasserstoffhaltigen Strömen, wie beispielsweise Flüssiggas (LPG), fertigen Kraftstoffen, Roh- und schweren Rückstandsölen, Erdgasasphalt, Bohrflüssigkeiten auf Ölbasis und wasserhaltigen Strömen, wie beispielsweise Bohrflüssigkeiten auf Wasserbasis.

12. Kohlenwasserstoffhaltige Zusammensetzung, umfassend Kohlenwasserstoffe und die Verbindung nach einem der Ansprüche 1 bis 5 oder die Zusammensetzung nach Anspruch 8.

13. Wasserhaltiger Strom, umfassend Wasser und die Verbindung nach einem der Ansprüche 1 bis 5 oder die Zusammensetzung nach Anspruch 8.

## Revendications

1. Composé de formule (I) : 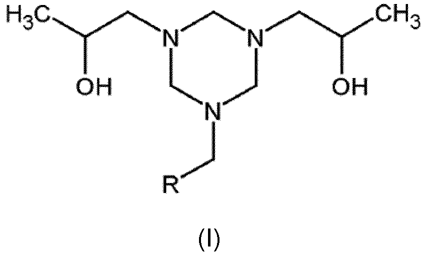 dans lequel
- R est un radical choisi parmi un groupe hydrocarbyle linéaire, ramifié ou cyclique comprenant éventuellement un ou plusieurs hétéroatomes, tels que des atomes d'oxygène, de soufre, d'halogène, de silicium, d'azote.

2. Composé selon la revendication 1, dans lequel R est choisi parmi : R¹-OH, R¹-[O-R²]ₚ-OH, benzyle éventuellement substitué, R¹-NR³R⁴, R⁵, R¹-[N(H)-R²]ₙ-NR³R⁴, R¹-[O-R²]ₚ-NR³R⁴, R¹-Q, R¹-[N(H)-R²]ₙ-Q, R¹-[O-R²]ₚ-Q, R¹-N(R³-Q)(R⁴-Q) dans lequel :
- R¹ est choisi parmi un radical alkyle ou alcényle, linéaire, ramifié ou cyclique ayant de 1 à 24 atomes de carbone, de préférence de 1 à 16 atomes de carbone, plus préférablement de 1 à 12 atomes de carbone, encore plus préférablement de 1 à 8 atomes de carbone ou parmi un radical aromatique éventuellement substitué par un ou plusieurs substituants choisis parmi alkyle en C1-C6, hydroxyle, alcoxy en C1-C6, ou alkylamine en C1-C6, de préférence un radical phényle substitué par alkyle en C1-C6 ;
- R² est un radical alkyle ou alcényle, linéaire, ramifié ou cyclique ayant de 1 à 24 atomes de carbone, de préférence de 1 à 16 atomes de carbone, plus préférablement de 1 à 12 atomes de carbone, encore plus préférablement de 1 à 8 atomes de carbone ;
- R³ et R⁴ sont, indépendamment l'un de l'autre, un atome d'hydrogène ou un radical alkyle ou alcényle, linéaire, ramifié ou cyclique ayant de 1 à 16 atomes de carbone, de préférence de 1 à 12 atomes de carbone, plus préférablement de 1 à 8 atomes de carbone, encore plus préférablement de 1 à 4 atomes de carbone ;
- R⁵ est un radical alkyle ou alcényle, linéaire, ramifié ou cyclique ayant de 1 à 30 atomes de carbone, de préférence de 1 à 24 atomes de carbone, plus préférablement de 1 à 16 atomes de carbone, encore plus préférablement de 1 à 12 atomes de carbone ;
- Q est un radical choisi parmi morpholine, pipéridine, pipérazine, ou le radical monovalent de formule : 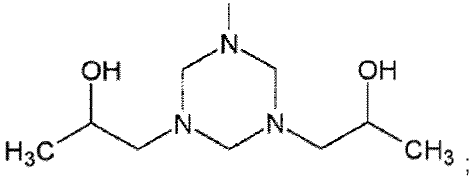
- le benzyle éventuellement substitué peut être un benzyle substitué par un ou plusieurs substituants choisis parmi alkyle en C1-C6, hydroxyle, alcoxy en C1-C6, alkylamine en C1-C6 ;
- n est un entier dans la plage de 1 à 10, de préférence de 1 à 6, plus préférablement de 1 à 4, encore plus préférablement de 1 à 2 ;
- p est un entier dans la plage de 1 à 10, de préférence de 1 à 6, plus préférablement de 1 à 4, encore plus préférablement de 1 à 2.

3. Composé selon la revendication 1 ou 2, dans lequel R est choisi parmi : R¹-OH, R¹-O-R²-OH, benzyle, R¹-NR³R⁴, R⁵, R¹-N(H)-R²-NR³R⁴, R¹-O-R²-O-R²-NR³R⁴, R¹-Q, R¹-N(H)-R²-Q, R¹-O-R²-O-R²-Q, R¹-N(R³-Q)₂ dans lequel :
- R¹ est choisi parmi un radical alkyle linéaire ou ramifié ayant de 1 à 16 atomes de carbone, de préférence de 1 à 12 atomes de carbone, plus préférablement de 1 à 8 atomes de carbone, encore plus préférablement de 1 à 4 atomes de carbone ou parmi un radical phényle substitué par alkyle en C1-C6 ;
- R² est un radical alkyle linéaire ou ramifié ayant de 1 à 16 atomes de carbone, de préférence de 1 à 12 atomes de carbone, plus préférablement de 1 à 8 atomes de carbone, encore plus préférablement de 1 à 4 atomes de carbone ;
- R³ et R⁴ sont, indépendamment l'un de l'autre, un atome d'hydrogène ou un radical alkyle linéaire ou ramifié ayant de 1 à 16 atomes de carbone, de préférence de 1 à 12 atomes de carbone, plus préférablement de 1 à 8 atomes de carbone, encore plus préférablement de 1 à 4 atomes de carbone ;
- R⁵ est un radical alkyle linéaire ou ramifié ayant de 1 à 24 atomes de carbone, de préférence de 1 à 16 atomes de carbone, plus préférablement de 1 à 12 atomes de carbone, encore plus préférablement de 1 à 8 atomes de carbone ;
- Q est un radical morpholine ou le radical monovalent de formule :

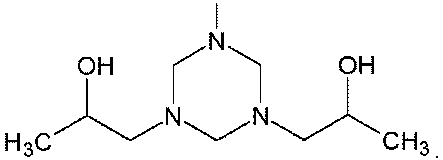

4. Composé selon l'une quelconque des revendications 1 à 3, dans lequel R est choisi parmi : R¹-OH, R¹-O-R²-OH, benzyle, R¹-NR³R⁴, R⁵, R¹-N(H)-R²-NR³R⁴, R¹-OR²-O-R²-NR³R⁴, R¹-Q, R¹-N(H)-R²-Q, R¹-O-R²-O-R²-Q, R¹-N(R³-Q)₂ dans lequel :
- R¹ est un radical alkyle linéaire ayant de 1 à 4 atomes de carbone ;
- R² est un radical alkyle linéaire ayant de 1 à 4 atomes de carbone ;
- R³ et R⁴ sont, indépendamment l'un de l'autre, un atome d'hydrogène ou un radical alkyle linéaire ayant de 1 à 4 atomes de carbone ;
- R⁵ est un radical alkyle linéaire ayant de 1 à 8 atomes de carbone ;
- Q est un radical morpholine ou le radical monovalent :

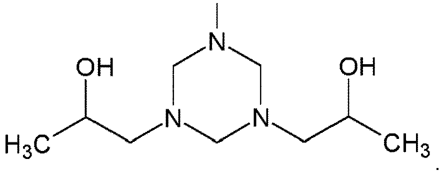

5. Composé selon l'une quelconque des revendications 1 à 4, répondant à l'une des formules suivantes :

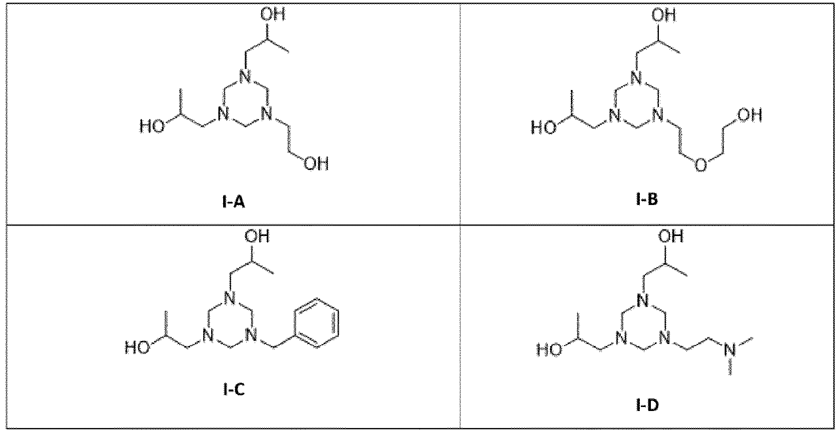

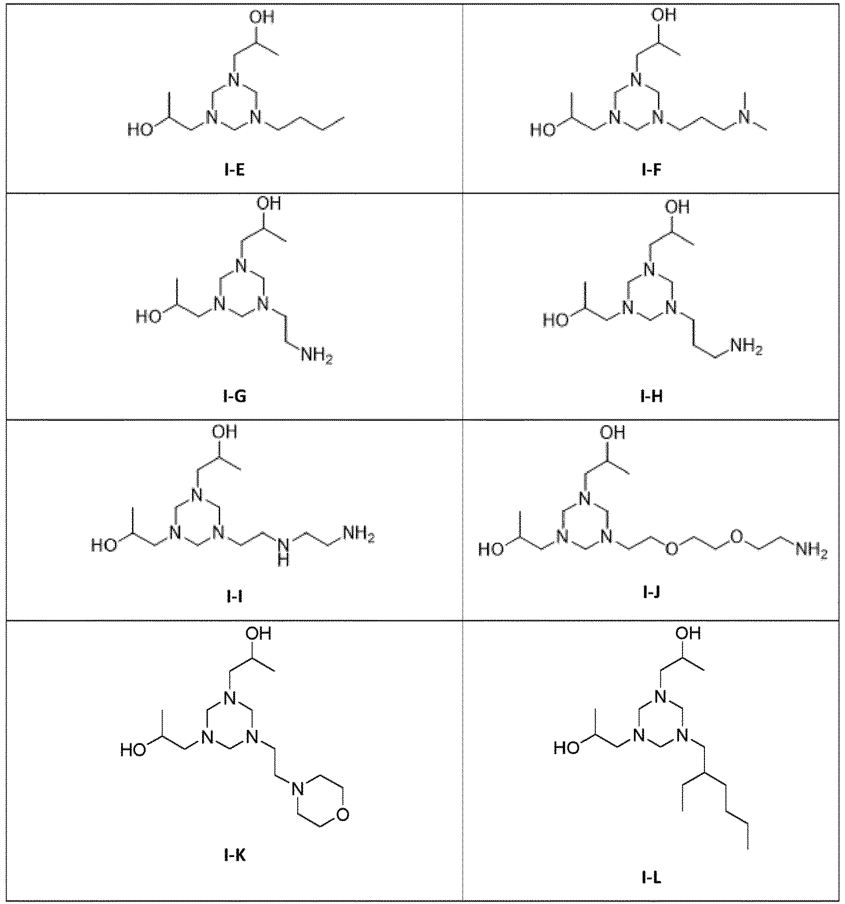

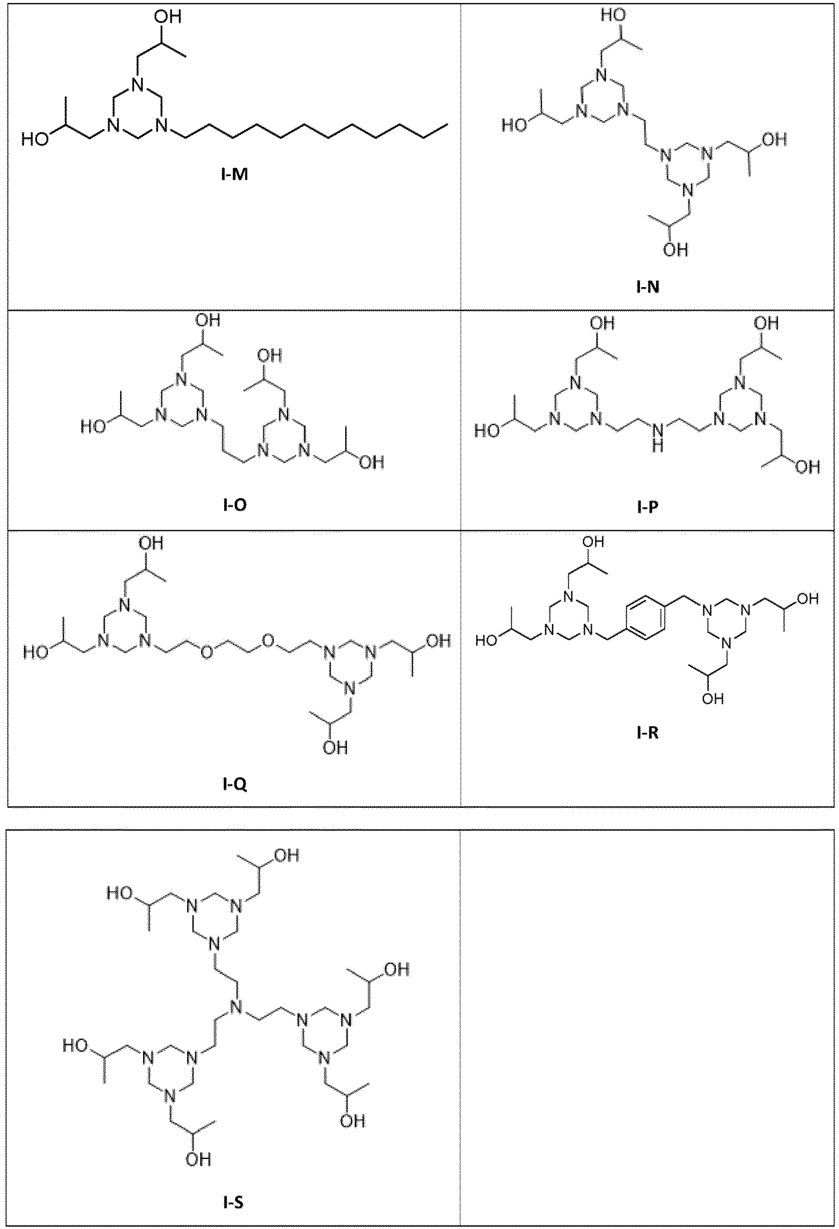

6. Procédé de fabrication du composé selon l'une quelconque des revendications 1 à 5, comprenant une étape de mise en contact d'au moins un bis(5-méthyloxazolidin-3-yl)méthane (BMOM) avec au moins une amine primaire.

7. Procédé selon la revendication 6, dans lequel l'étape de mise en contact est conduite avec un rapport molaire BMOM:amines primaires dans la plage de 1:5 à 5:1, de préférence de 1:1 à 3:1.

8. Composition comprenant :
- de 20 à 98 % en poids d'au moins un composé selon l'une quelconque des revendications 1 à 5, et
- de 2 à 80 % en poids de solvant(s),
par rapport au poids total de la composition.

9. Utilisation du composé selon l'une quelconque des revendications 1 à 5 ou de la composition selon la revendication 8, pour piéger le sulfure d'hydrogène et les mercaptans dans des flux choisis parmi des flux hydrocarbonés et des flux contenant de l'eau.

10. Utilisation selon la revendication 9, dans laquelle le composé est ajouté en une quantité d'au moins 1 % en poids, par rapport au poids total de sulfure d'hydrogène et de mercaptans dans le flux.

11. Utilisation selon la revendication 9 ou 10, dans laquelle les flux sont choisis parmi des flux contenant des hydrocarbures tels que du gaz de pétrole liquéfié (GPL), des carburants finis, des pétroles bruts et résiduels lourds, de l'asphalte de gaz naturel, des fluides de forage à base de pétrole et des flux contenant de l'eau tels que des fluides de forage à base d'eau.

12. Composition contenant des hydrocarbures comprenant des hydrocarbures et le composé selon l'une quelconque des revendications 1 à 5 ou la composition selon la revendication 8.

13. Flux contenant de l'eau comprenant de l'eau et le composé selon l'une quelconque des revendications 1 à 5 ou la composition selon la revendication 8.
